(19) **Europäisches Patentamt / European Patent Office / Office européen des brevets**

(11) **EP 2 581 431 B1**

(12) **EUROPEAN PATENT SPECIFICATION**

(45) Date of publication and mention
of the grant of the patent:
**06.03.2019 Bulletin 2019/10**

(21) Application number: **11792585.9**

(22) Date of filing: **13.06.2011**

(51) Int Cl.:
*C09K 3/30* *(2006.01)*    *B65D 83/34* *(2006.01)*
*B65D 83/36* *(2006.01)*    *A61Q 19/00* *(2006.01)*
*A61K 8/31* *(2006.01)*    *A61K 8/39* *(2006.01)*
*A61K 8/44* *(2006.01)*    *A61K 8/69* *(2006.01)*
*A61K 8/86* *(2006.01)*    *A61K 8/04* *(2006.01)*

(86) International application number:
**PCT/JP2011/063516**

(87) International publication number:
**WO 2011/155630 (15.12.2011 Gazette 2011/50)**

(54) **AEROSOL COMPOSITION**

AEROSOLZUSAMMENSETZUNG

COMPOSITION D'AÉROSOL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **22.03.2011   JP 2011062444
11.06.2010   JP 2010134565**

(43) Date of publication of application:
**17.04.2013   Bulletin 2013/16**

(73) Proprietor: **Daizo Corporation
Osaka-shi
Osaka 552-0013 (JP)**

(72) Inventors:
• **TERAMOTO, Keiichiro
Kyoto-shi
Kyoto 613-0916 (JP)**

• **OKANO, Fuminori
Sashima-gun
Ibaraki 306-0314 (JP)**
• **WAGAMITSU, Atsushi
Sashima-gun
Ibaraki 306-0314 (JP)**

(74) Representative: **Gille Hrabal
Brucknerstrasse 20
40593 Düsseldorf (DE)**

(56) References cited:
**WO-A1-02/083321      JP-A- 2 255 890
JP-A- 2003 335 629     JP-A- 2009 227 662
US-A1- 2006 269 484**

**Description**

TECHNICAL FIELD

The present invention relates to an aerosol composition, in particular to an aerosol composition which is high in fire safety and assures easy emulsification of an aqueous concentrate and a liquefied gas.

BACKGROUND ART

Patent Document 1 discloses a process for preparing an aerosol composition by emulsifying an aqueous concentrate and a liquefied petroleum gas in an aerosol container. When this aerosol composition is sprayed in the air, the liquefied petroleum gas is held in the aqueous concentrate for a long period of time by emulsification with the aqueous concentrate and the aqueous concentrate is frozen. In Patent Document 2, an aerosol composition prepared by emulsification of an aqueous concentrate and a liquefied gas having a specific boiling point in an aerosol container is disclosed, and when this aerosol composition is sprayed, foams emitting a foam-cracking sound are formed. Further, in Patent Document 3, an aerosol composition prepared by emulsification of an aqueous concentrate and a liquefied gas in an aerosol container is disclosed, and a spray of independent foams in the form of soap bubble is obtained.

Patent Document 4 discloses medicinal compositions and devices using them comprising a propellant and at least one medicinally active compound, said propellant comprising at least one fluoroolefin having at least two but less than seven carbon atoms. Suitable fluoroolefins include HFO-1234ze or HFO-1234yf. Preferably, the compositions comprise at least about 50 % by weight of propellant based on the total weight of the composition. A water-containing composition is not disclosed in the document.

Patent Document 5 discloses an aerosol composition comprising a water-containing stock solution containing an active component and a surface active agent, and, optionally, an alcohol and a water-soluble silicone oil, and a propellant. The aerosol composition of Patent Document 5 comprises not less than 50 % by weight of water.

Patent Document 6 discloses a pest control aerosol sprayer comprising a pressure-tight container receiving therein a noxious insect behavioral inhibitor, of which an active ingredient is HFC-152a acting both as a propellant and a refrigerant. Patent Document 6 also discloses other alternatives for chlorofluorocarbon including hydrofluoroolefin having very low inflammability, which cannot be emulsified with water.

PRIOR ART DOCUMENTS

Patent Documents

Patent Document 1: JP 3439672 B2

Patent Document 2: JP 4173034 B2

Patent Document 3: JP 4098093 B2

Patent Document 4: US 2006/269484 A1

Patent Document 5: JP 2-255890 A

Patent Document 6: JP -2009-227662-A corresponding to EP-2-245-927-A1

DISCLOSURE OF THE INVENTION

PROBLEM TO BE SOLVED BY THE INVENTION

However, there is a problem that an emulsifying efficiency is not good because in order to emulsifying an aqueous concentrate and a liquefied gas, after filling the both in an aerosol container, the container is shaken up and down to mix them in the container. Concretely, it is necessary that in a production line, after filling an aqueous concentrate and a liquefied gas, the mixture is once taken out from the production line, and an aerosol container is set on a shaking device for emulsification, and then the emulsified mixture is returned to the production line again. Thus, there is a problem that efficiency is not good. Also, there is a problem with safety because a liquefied gas is inflammable.

The present invention was made in the light of the above-mentioned problems, and an object of the present invention is to provide an aerosol composition which is high in fire safety and assures easy emulsification of an aqueous concentrate and a liquefied gas.

MEANS TO SOLVE THE PROBLEM

The aerosol composition of the present invention is one comprising 10 to 60 wt% of an aqueous concentrate comprising a surfactant and water and 40 to 90 wt% of a liquefied gas, and obtained by emulsifying the aqueous concentrate and the liquefied gas, wherein a content of water in the aqueous concentrate is 40 to 88 wt.%, wherein the liquefied gas comprises a heavy liquefied gas (a) having a liquid density at 20°C of from 1.15 to 1.30 (g/ml) and a light liquefied gas (b) having a liquid density at 20 °C of from 0.50 to 0.70 (g/ml), wherein the heavy liquefied gas is hydrofluoroolefin, and wherein the aerosol composition is obtained by emulsifying the aqueous concentrate, the hydrofluoroolefin and the light liquefied gas.

It is preferable that a content of the heavy liquefied gas (a) in the aerosol composition is 5 wt% or more.

It is preferable that a liquid density of the aqueous concentrate is from 0.90 to 1.10 (g/ml).

It is preferable that when the aerosol composition is sprayed, sherbet which is at least partly frozen is formed, and when the composition is sprayed against a 5 cm high flame located apart by 15 cm, elongation of the flame is 50 cm or less.

It is preferable that foam emitting a foam-cracking sound is formed when the aerosol composition is sprayed, and a height of a flame when the foam is ignited is 35 cm or less.

It is preferable that many independent foams in the form of soap bubble are formed when the aerosol composition is sprayed, and a falling speed of the foams is from 0.015 to 0.2 m/sec.

It is preferable that the aqueous concentrate comprises an ionic surfactant and/or an ionic resin.

EFFECT OF THE INVENTION

According to the present invention, an aerosol composition which is high in fire safety and assures easy emulsification of an aqueous concentrate and a liquefied gas can be provided.

EMBODIMENT FOR CARRYING OUT THE INVENTION

The aerosol composition of the present invention is one comprising 10 to 60 wt% of an aqueous concentrate comprising a surfactant and water and 40 to 90 wt% of a liquefied gas, wherein a content of water in the aqueous concentrate is 40 to 99 wt.%, which obtained by emulsifying the aqueous concentrate and the liquefied gas, wherein the liquefied gas comprises a heavy liquefied gas (a) having a liquid density at 20°C of from 1.15 to 1.30 (g/ml) and a light liquefied gas (b) having a liquid density at 20 °C of from 0.50 to 0.70 (g/ml), wherein the heavy liquefied gas is a hydrofluoroolefin.

The aerosol composition of the present invention can be one which, when sprayed, forms sherbet being at least partly frozen, forms foam emitting a foam-cracking sound, or forms many independent foams in the form of soap bubble.

The above-mentioned many independent foams in the form of soap bubble mean foams which independently form a single membrane comprising an aqueous concentrate and being wholly in contact with outside air (the foam forms a single sphere) or a plurality of independent foams in the form of soap bubble coming into contact with each other and being in contact with outside air. The independent foams in the form of soap bubble are characterized in that adhesion of suspended matters in a space is improved because after spraying, an area occupied by the foams in a space is wide and an area of the membrane being in contact with outside air is wide as compared with sprayed aerosol composition which comprises an aqueous concentrate of the same mass and is not in the form of soap bubble.

It is preferable that a diameter of the above-mentioned individual foams in the form of soap bubble is from 0.1 to 10 mm, further preferably from 0.2 to 5 mm. When the diameter of the foam is less than 0.1 mm, there is a tendency that a floating time of the foams in a space is long, suspended matters in a space is hardly removed, sprayed foams are hardly observed with naked eyes, and further, a user aspirates foams easily. When the diameter of the foam is more than 10 mm, there is a tendency that suspended matters in a space cannot be adhered efficiently because an area of the foam membrane being in contact with outside air is decreased.

A falling speed of foams in the form of soap bubble is preferably from 0.015 to 0.2 m/sec. When the falling speed of foams in the form of soap bubble is slower than 0.015 m/sec, there is a tendency that a floating time is long and the foams lose directional property and float in the air. When the falling speed is faster than 0.2 m/sec, there is a tendency that a falling time is shorter and adhesion of suspended matters is lowered. Particularly, a preferred falling speed is from 0.020 to 0.15 m/sec from the point that suspended matters in a space can be adhered efficiently.

In the above-mentioned aqueous concentrate, a surfactant is contained in water for emulsification with the liquefied gas, and a resin, water-soluble polymer, active ingredient, alcohols, oils, powder, etc. can be contained therein. A liquid density of the aqueous concentrate is preferably from 0.90 to 1.10 (g/ml). In the case where the liquid density of the aqueous concentrate is out of the range of from 0.90 to 1.10 (g/ml), emulsification with the liquefied gas tends to be hardly carried out.

Examples of the surfactant are nonionic surfactants having HLB of 10 to 19, preferably 11 to 18, for example, POE sorbitan fatty acid esters such as POE sorbitan monolaurate, POE sorbitan monostearate, POE sorbitan monooleate, POE sorbitan monopalmitate and POE sorbitan monoisostearate; polyoxyethylene polyoxypropylene alkyl ethers such as POE POP cetyl ether and POE POP decyl tetradecyl ether; polyoxyethylene glycerin fatty acid ester such as POE glyceryl monostearate; polyoxyethylene lanolin alcohol such as POE lanolin alcohol; polyoxyethylene hydrogenated castor oil such as POE hydrogenated castor oil; polyoxyethylene alkyl ethers such as POE cetyl ether, POE stearyl ether, POE oleyl ether, POE lauryl ether, POE behenyl ether, POE octyl dodecyl ether, POE isocetyl ether and POE isostearyl ether; polyethylene glycol fatty acid esters such as polyethylene glycol monostearate; polyglycerin fatty acid esters such as hexaglyceryl monolaurate, hexaglyceryl monomyristate, pentaglyceryl monolaurate, pentaglyceryl monomyristate, pentaglyceryl monooleate, pentaglyceryl monostearate, decaglyceryl monolaurate, decaglyceryl monomyristate, decaglyceryl monostearate, decaglyceryl monoisostearate, decaglyceryl monooleate and decaglyceryl monolinolate; polyoxyethylene glycerin fatty acid esters such as polyoxyethylene glyceryl monooleate; and polyoxyethylene sorbitan fatty acid esters such as POE sorbitan monolaurate, POE sorbitan tetrastearate and POE sorbitan tetraoleate. When HLB is less than 10, a liquefied gas is apt to be in continuous phase, and when HLB is more than 19, a liquefied gas tends to be hardly emulsified. Other examples of the surfactant are anionic surfactants such as fatty acid soap, alkyl sulfate, polyoxyethylene alkyl ether sulfate, alkyl phosphate and polyoxyethylene alkyl ether phosphate; silicone surfactants such as polyoxyethylene-methyl polysiloxane copolymer, polyoxypropylene-methyl polysiloxane copolymer and poly(oxyethylene-oxypropylene)-methyl polysiloxane copolymer; natural surfactants such as sodium surfactin, cyclodextrin and hydrogenated enzymatically modified soybean lecithin; amino acid surfactants such as N-acylglutamic acid salts such as triethanolamine N-cocoyl-L-glutamate, potassium N-cocoyl-L-glutamate, sodium N-cocoyl-L-glutamate, triethanolamine N-lauroyl-L-glutamate, potassium N-lauroyl-L-glutamate, sodium N-lauroyl-L-glutamate, potassium N-myristoyl-L-glutamate, sodium N-myristoyl-L-glutamate and sodium N-stearoyl-L-glutamate, N-acyl glutamic acids such as N-cocoyl-L-glutamic acid, N-lauroyl-L-glutamic acid and N-stearoyl-L-glutamic acid, N-acyl glycine salts such as potassium N-cocoyl glycinate and sodium N-cocoyl glycinate and N-acyl alanine salts such as triethanolamine N-cocoyl-DL-alaninate; and mixtures thereof. From the viewpoint of especially excellent emulsification stability with a heavy liquefied gas, it is preferable to use nonionic surfactants, silicone surfactants and amino acid surfactants. Moreover, in the case of the aerosol composition forming many independent foams in the form of soap bubble, it is preferable to use nonionic surfactants because emulsification with a liquefied gas including a heavy liquefied gas is easy and a size of the foams is easily adjusted, and a combination use thereof with a surfactant having ionicity such as an anionic, cationic, amphoteric or amino acid surfactant is preferred from the point of easily adhering and removing suspended matters such as house dusts and pollen.

An amount of the above-mentioned surfactant is preferably from 0.1. to 20 wt%, further preferably from 0.2 to 15 wt% in the aqueous concentrate. When the amount of the surfactant is less than 0.1 wt%, emulsification stability of the aqueous concentrate and the liquefied gas tends to be decreased, and when the amount of the surfactant is more than 20 wt%, foams are apt to remain on an adhered surface and impression from use is not good. Especially in the case of the aerosol composition in which the sprayed aerosol composition forms foams in the form of soap bubble, the amount of the surfactant is preferably from 1 to 20 wt%, further preferably from 2 to 15 wt% in the aqueous concentrate.

The above-mentioned water is a main solvent of the aqueous concentrate and enhances fire safety. Examples of water are purified water, ion exchange water, physiological saline, deep sea water and the like.

An amount of water is from 40 to 99 wt%, further preferably from 50 to 97 wt% in the aqueous concentrate. When the amount of water is less than 40 wt%, there is a tendency that emulsification is hardly achieved and fire safety is hardly obtained. When the amount of water is more than 99 wt%, there is a tendency that a necessary amount of surfactant for emulsifying with the liquefied gas is hardly added.

The above-mentioned resin has a function of assisting the emulsification of the aqueous concentrate and the liquefied gas and improving emulsification stability of the aerosol composition by adjusting the density of the aqueous concentrate. Particularly when using an ionic resin, it is easy to obtain an effect of adhering and removing suspended matters such as house dusts and pollen by adsorption thereof.

Examples of the above-mentioned resin are anionic resins such as (acrylates/alkyl acrylate/ethyl methacrylate amine oxide) copolymer; cationic resins such as vinyl pyrrolidone/N,N-dimethylaminoethyl methacrylate copolymer diethyl sulfate, methyl vinyl imidazolium chloride/vinyl pyrrolidone copolymer and methyl vinyl imidazolium/vinyl pyrrolidone copolymer methyl sulfate; acrylic acid-based amphoteric resins such as (octylacrylamide/ hydroxypropyl acrylate / butylaminoethyl methacrylate) copolymer; nonionic resins such as polyvinyl pyrrolidone, vinyl acetate/vinyl pyrrolidone copolymer, polyvinyl caprolactam, N-vinyl pyrrolidone/methacrylamide/N-vinyl imidazole copolymer, copolymers of (diacetone acrylamide/ester of aliphatic alcohol having 4 to 18 carbon atoms and acrylic acid or methacrylic acid/at least one of acrylic acid, methacrylic acid and itaconic acid/at least one of acrylic ester or methacrylic acid ester having 1 to 3 carbon atoms) and copolymers of (dimethyl acrylamide/hydroxyethyl acrylate/methoxyethyl acrylate); and the like. When using especially ionic resins such as anionic resins, cationic resins and amphoteric resins, it is easy to obtain an effect of adhering and removing suspended matters such as house dusts and pollen by adsorption thereof.

A solid content of the above-mentioned resin is preferably from 0.01 to 5 wt%, further preferably from 0.1 to 3 wt% in the aqueous concentrate. When the content of the resin is less than 0.01 wt%, there is a tendency that the above-mentioned effects are hardly obtained. When the content of the resin is more than 5 wt%, there is a tendency that after having fallen on a tatami mat, floor, carpet and the like, the fallen composition is apt to adhere thereto and is hardly removed.

The above-mentioned water-soluble polymer has a function of adjusting a viscosity of the aqueous concentrate to increase emulsification stability with the liquefied gas and even after spraying in the air, keeping an emulsified state to increase a cracking sound and allow a sprayed aerosol composition to be easily frozen and enlarging a size of foams in the form of soap bubble.

Examples of the above-mentioned water-soluble polymer are gums such as xanthan gum, carrageenan, acacia gum, tragacanth gum, cationic guar gum, guar gum, gellan gum and locust bean gum; cellulose polymers such as hydroxyethyl cellulose, hydroxypropyl cellulose, hydroxypropyl methyl cellulose, carboxymethyl-cellulose sodium, cellulose nitrate and crystalline cellulose; dextran, carboxymethyl-dextran sodium, dextrine, pectin, starch, cornstarch, wheat starch, sodium alginate, denatured potato starch, sodium hyaluronate, polyvinyl alcohol, polyvinyl pyrrolidone, carboxyvinyl polymer and the like.

An amount of the above-mentioned water-soluble polymer is preferably from 0.01 to 5 wt%, further preferably from 0.05 to 3 wt% in the aqueous concentrate. When the amount of the water-soluble polymer is less than 0.01 wt%, the above-mentioned effect tends to be hardly obtained, and when the amount of the water-soluble polymer exceeds 5 wt%, there is a tendency that the viscosity of the aqueous concentrate becomes too high and it takes a long time for emulsification with the liquefied gas.

The viscosity of the aqueous concentrate is preferably from 1 to 20,000 (mPa·s at 20°C), further preferably from 3 to 10,000 (mPa·s). When the viscosity of the aqueous concentrate is less than 1 (mPa·s), emulsification stability tends to be decreased, and when the viscosity of the aqueous concentrate is more than 20,000 (mPa·s), there is a tendency that the aqueous concentrate is hardly emulsified with the low viscosity liquefied gas.

Examples of the active ingredient are anti-itchings such as crotamiton and d-camphor, and antiphlogistic anodynes such as methyl salicylate, indomethacin, piroxicam, felbinac and ketoprofen; antifungal agents such as oxiconazole, clotrimazole, sulconazole, bifonazole, miconazole, isoconazole, econazole, tioconazole, butenafine, and hydrochlorides, nitrates and acetates thereof; astringents such as zinc oxide, aluminum hydroxy allantoinate, tannic acid, citric acid and lactic acid; anti-inflammatory agents such as allantoin, glycyrrhetinic acid, dipotassium glycyrrhizate and azulene; local anesthetics such as dibucaine hydrochloride, tetracaine hydrochloride, lidocaine and lidocaine hydrochloride; antihistamines such as diphenhydramine, diphenhydramine hydrochloride and chlorpheniramine maleate; sterilization and disinfection agents such as paraoxybenzoic acid, sodium benzoate, phenoxyethanol, benzalkonium chloride, benzethonium chloride and chlorhexidine chloride; refrigerants such as 1-menthol and camphor; humectants such as ethylene glycol, propylene glycol, glycerin, 1,3-butylene glycol, collagen, xylitol, sorbitol, hyaluronic acid, karonin acid, sodium lactate, dl-pyrrolidone carboxylate, keratin, lecithin and urea; deodorants such as lauric acid methacrylate, methyl benzoate, methyl phenyl acetate, geranyl crotonate, acetophenone myristate, benzyl acetate, benzyl propionate and green tea extract; vermin repellents such as N,N-diethyl-m-toluamide (Deet) and capronic acid diethylamide; insecticides such as phthalthrin, allethrin, permethrin, cismethrin, proparthrin, resmethrin, d-phenothrin, tefluthrin and benfluralin and effect enhancer such as synepirin, piperonyl butoxide and octachloro dipropyl ether; ultraviolet absorbers such as p-methoxy cinnamic acid 2-ethylhexyl, ethylhexyl triazone and oxybenzone; ultraviolet scattering agents such as zinc oxide and titanium oxide; vitamins such as retinol, retinol acetate, retinol palmitate, calcium pantothenate, ascorbic acid, sodium ascorbate, dl-$\alpha$-tocopherol, tocopherol acetate, tocopherol and mixtures thereof; antioxidants such as ascorbic acid, $\alpha$-tocopherol and dibutyl hydroxytoluene; extraction liquids such as peony extract, loofah extract, rose extract, lemon extract, aloe extract, iris root extract, eucalyptus extract, sage extract, tea extract, sea weed extract, placenta extract and silk extract; skin lightening agents such as arbutin and kojic acid; perfumes such as natural perfumes and synthetic perfumes, and the like.

An amount of the active ingredient is from 0.05 to 10 wt%, preferably from 0.1 to 8 wt% in the aqueous concentrate. When the amount of the active ingredient is less than 0.05 wt%, there is a tendency that the effect of the active ingredient is not exhibited sufficiently, and when the amount of the active ingredient is more than 10 wt%, a concentration of the active ingredient becomes too high, which has an adverse effect on a human body depending on kind of an active ingredient.

The above-mentioned alcohols are used for the purposes of using as a solvent for dissolving the active ingredient being hardly dissolved in water, adjusting a cracking sound and a freezing degree when spraying, and further accelerating separation of foams and making formation of many foams in the form of soap bubble easy when spraying.

Examples of the alcohols are mono-valent alcohols having 2 to 3 carbon atoms such as ethanol and isopropanol, di- and tri-valent polyols such as ethylene glycol, propylene glycol, 1,3-butylene glycol, diethylene glycol, dipropylene glycol and glycerin, and the like.

An amount of the alcohols is preferably from 0.1 to 50 wt%, further preferably from 0.3 to 45 wt% in the aqueous concentrate. When the amount of the alcohols is less than 0.1 wt%, there is a tendency that the above-mentioned effects are hardly obtained, and when the amount of the alcohols is more than 50 wt%, there is a tendency that the aqueous concentrate and the liquefied gas are hardly emulsified.

When preparing an aerosol composition forming foams emitting a foam-cracking sound at praying, the amount of the alcohols is preferably from 15 to 30 wt% in the aqueous concentrate. When the amount of the alcohols is less than 15 wt%, there is a tendency that the sprayed aerosol composition is easily frozen, and when the amount of the alcohols is more than 30 wt%, a foam-cracking sound tends to be smaller.

When preparing an aerosol composition forming at least partly frozen sherbet at spraying, the amount of the alcohols is preferably from 0.1 to 15 wt% in the aqueous concentrate. When the amount of the alcohols is less than 0.1 wt%, there is a tendency that the above-mentioned effects are hardly obtained, and when the amount of the alcohols is more than 15 wt%, the sprayed aerosol composition tends to be hardly frozen.

When preparing an aerosol composition forming many independent foams in the form of soap bubble at spraying, the amount of the alcohols is preferably from 20 to 50 wt% in the aqueous concentrate. When the amount of the alcohols is less than 20 wt%, there is a tendency that separation of foams is not good, and when the amount of the alcohols is more than 50 wt%, there is a tendency that the sprayed aerosol composition is not foamed and is formed into a mist.

The above-mentioned oils are used for the purposes of adjusting an emulsified condition of the aqueous concentrate and the liquefied gas, removing oil from a targeted object, or making infiltration into a targeted object easy.

Examples of the oils are hydrocarbon oils such as liquid paraffin, squalene, squalane and isoparaffin; ester oils such as diisopropyl adipate, isopropyl myristate, isopropyl palmitate, cetyl octanoate, octyl dodecyl myristate, butyl stearate, myristyl myristate, decyl oleate, cetyl lactate, isocetyl stearate, cetostearyl alcohol, diisobutyl adipate, diisopropyl sebacate, diethoxyethyl succinate, diisostearyl malate and methylpentanediol dineopentanoate; silicone oils such as methyl polysiloxane, octamethylcyclotetrasiloxane, decamethylcyclopentasiloxane, dodecamethylcyclohexasiloxane, methylcyclopolysiloxane, tetrahydrotetramethylcyclotetrasiloxane, octamethyltrisiloxane, decamethyltetrasiloxane, methylhydrogenpolysiloxane, methylphenylpolysiloxane and cyclopentasiloxane; fatty acids such as lauric acid, myristic acid, palmitic acid, stearic acid, behenic acid, oleic acid and isostearic acid; higher alcohols such as lauryl alcohol, myristyl alcohol, cetyl alcohol, stearyl alcohol, behenyl alcohol and lanolin alcohol; fats and fatty oils such as avocado oil, macadamia nut oil, shea butter, olive oil and camellia oil; waxes such as bees wax and lanolin wax; and the like. It is preferable to use ester oils such as methylpentanediol dineopenanoate especially when preparing the aerosol composition forming foams emitting a foam-cracking sound at spraying since the liquefied gas is kept easily in the sprayed aerosol composition and the foam-cracking sound can be made large.

An amount of the oil is preferably from 0.1 to 20 wt%, further preferably from 0.5 to 15 wt% in the aqueous concentrate. When the amount of the oil is less than 0.1 wt%, there is a tendency that an effect to be produced by adding the oil is hardly obtained, and when the amount of the oil is more than 20 wt%, there is a tendency that drying characteristic is lowered and impression from use is decreased.

The above-mentioned powder is used as an emulsification accelerator for the purposes of making emulsification of the aqueous concentrate and the liquefied gas easy and improving emulsification stability.

Examples of the powder are talc, zinc oxide, kaolin, mica, magnesium carbonate, calcium carbonate, zinc silicate, magnesium silicate, aluminum silicate, calcium silicate, silica, zeolite, ceramic powder, boron nitride and the like.

An amount of the above-mentioned powder is preferably from 0.01 to 5 wt%, further preferably from 0.03 to 3 wt% in the aqueous concentrate. When the amount of the powder is less than 0.01 wt%, there is a tendency that the above-mentioned effects are hardly obtained. When the amount of the powder is more than 5 wt%, there is a tendency that clogging easily arises in a spraying path, especially in a spray nozzle.

Especially the use of a powder having an average primary particle size of 5 to 25 nm and having a hydrophilic surface is preferred since emulsification of the aqueous concentrate and the liquefied gas is easy.

The aqueous concentrate used in the present invention is prepared by dissolving a surfactant, a water soluble polymer to be added as needed, and the like in water and alcohols. In the aqueous concentrate, according to the necessity, oil may be emulsified and a powder may be dispersed.

An amount of the aqueous concentrate is from 10 to 60 wt%, preferably from 15 to 50 wt% in the aerosol composition. When the amount of the aqueous concentrate is less than 10 wt%, emulsification hardly proceeds, and when the amount of the aqueous concentrate is more than 60 wt%, there is a tendency that a cracking sound of the sprayed aerosol composition becomes small, the sprayed aerosol composition is hardly frozen and foams in the form of soap bubble are hardly formed.

The above-mentioned liquefied gas is a liquid in the aerosol container, and is emulsified with the aqueous concentrate to form an emulsified composition.

The above-mentioned liquefied gas comprises the heavy liquefied gas (a) having a liquid density at 20°C of from 1.15 to 1.30 (g/ml), and is used by mixing with the light liquefied gas (b) having a liquid density at 20°C of from 0.50 to 0.70 (g/ml).

The above-mentioned heavy liquefied gas is a safe liquefied gas because if it is sprayed against a flame, no flame propagation is recognized. By emulsification with the aqueous concentrate, the heavy liquefied gas easily remains not only in the composition at spraying but also in the sprayed aerosol composition, thereby increasing fire safety. Especially even in the case of using a light liquefied gas having high inflammability, by emulsifying the aqueous concentrate, the heavy liquefied gas and the light liquefied gas, the heavy liquefied gas is cooled due to heat absorption by evaporation of the light liquefied gas immediately after the spraying and easily remains in the sprayed aerosol composition, thereby inhibiting inflammability of the sprayed aerosol composition and increasing fire safety. Further, since the light liquefied gas and the heavy liquefied gas are dissolved into one liquid, a liquid density of the liquefied gas can be adjusted. By decreasing a difference in a liquid density between the liquefied gas and the aqueous concentrate, the liquefied gas is easily emulsified with the aqueous concentrate and is hardly separated from the aqueous concentrate. It is more preferable that the liquid density of the heavy liquefied gas is from 1.15 to 1.25 (g/ml). When the liquid density of the heavy liquefied gas is less than 1.15 (g/ml), there is a tendency that an effect obtained by decreasing a difference in a liquid density between the liquefied gas and the aqueous concentrate by combination use of the light liquefied gas is hardly obtained, and an effect that the heavy liquefied gas is easily emulsified with the light liquefied gas and the aqueous concentrate is hardly obtained. When the liquid density of the heavy liquefied gas is more than 1.30 (g/ml), there is a tendency that a sedimentation velocity of the heavy liquefied gas in the aerosol container becomes fast and the heavy liquefied gas is hardly emulsified with the light liquefied gas and the aqueous concentrate, and also, there is a problem that the heavy liquefied gas is easily separated from the aqueous concentrate with a lapse of time.

The above-mentioned heavy liquefied gas is hydrofluoroolefin , and especially trans-1,3,3,3-tetrafluoropropa-1-ene (liquid density: 1.19 g/ml, HFO-1234ze) and trans-2,3,3,3-tetrafluoropropa-1-ene (liquid density: 1.19 g/ml, HFO-1234yf) are preferable.

An amount of the heavy liquefied gas is preferably from 5 to 90 wt%, further preferably from 10 to 87 wt% in the aerosol composition. When the amount of the heavy liquefied gas is less than 5 wt%, an effect of inhibiting inflammability tends to be insufficient, and when the amount of the heavy liquefied gas is more than 90 wt%, the heavy liquefied gas tends to be hardly emulsified and easily separated.

When preparing the aerosol composition forming foams emitting a foam-cracking sound at spraying, by adding the heavy liquefied gas in an amount of not less than 10 wt% in the aerosol composition, the heavy liquefied gas easily remains in the foams and fire safety is increased, and when setting fire directly to the foams, a height of flame is not more than 35 cm and firing time can be shortened to 10 seconds or less. Especially by adding the heavy liquefied gas in an amount of not less than 40 wt% in the aerosol composition, even if inflammables such as alcohol are added in the aqueous concentrate, a height of flame is not more than 5 cm when setting fire directly to the foams, and firing time can be as very short as 3 seconds or less.

When preparing an aerosol composition forming sherbet which is at least partly frozen at spraying, by adding the heavy liquefied gas in an amount of not less than 10 wt% in the aerosol composition, elongation of a flame length can be 45 cm or less even if a liquid phase of aerosol composition is sprayed directly to the flame. Especially by adding the heavy liquefied gas in an amount of not less than 40 wt% in the aerosol composition, even if the inflammable light liquefied gas is added in an amount of not less than 40 wt% in the aerosol composition, increase of a flame length can be prevented. Thereby, even if a valve without a vapor tap for introducing a gaseous phase of the liquefied gas is used, fire safety can be secured. Therefore, a liquid phase of the liquefied gas can be contained in the sprayed aerosol composition at high concentration, and the sprayed aerosol composition can be easily frozen.

When preparing an aerosol composition forming many independent foams in the form of soap bubble at spraying, by adding the heavy liquefied gas in an amount of not less than 10 wt% in the aerosol composition, the foams in the form of soap bubble fall easily and a scattering time in a space can be adjusted. Further, by adding the heavy liquefied gas in an amount of not less than 40 wt% in the aerosol composition, increase of a flame length can be prevented even if the inflammable light liquefied gas is added in an amount up to 40 wt% in the aerosol composition.

The above-mentioned light liquefied gas is used for the purposes of making the emulsification of the aqueous concentrate and the heavy liquefied gas easy, adjusting the pressure of the aerosol composition, adjusting a foam-cracking sound of the sprayed aerosol composition, adjusting the sprayed aerosol composition to be easily frozen, and adjusting a falling speed of foams in the form of soap bubble. It is more preferable that the liquid density of the light liquefied gas is from 0.50 to 0.60 (g/ml). When the liquid density of the light liquefied gas is less than 0.50 (g/ml), the light liquefied gas tends to be hardly emulsified with the heavy liquefied gas and the aqueous concentrate, and when the liquid density of the light liquefied gas is more than 0.70 (g/ml), there is a tendency that an effect of making small a difference in a liquid density between the liquefied gas and the aqueous concentrate is hardly obtained, and the light liquefied gas is hardly emulsified with the heavy liquefied gas and the aqueous concentrate and is easily separated from the aqueous concentrate with a lapse of time. The light liquefied gas is not limited particularly as far as its liquid density is within the above-mentioned range. Examples of preferred light liquefied gas are propane (liquid density: 0.501 g/ml), normal butane (liquid density: 0.579 g/ml), isobutane (liquid density: 0.557 g/ml), a liquefied petroleum gas which is a mixture thereof, dimethyl ether (liquid density: 0.661 g/ml), and a gas mixture of a liquefied petroleum gas and dimethyl ether, in that the liquid density of the light liquefied gas is adjusted easily and the pressure of the aerosol composition is adjusted easily. The liquid density of the light liquefied gas may be adjusted by adding hydrocarbon such as normal pentane or isopentane to the light liquefied gas.

When adding the light liquefied gas, its amount is preferably from 3 to 80 wt%, further preferably from 5 to 70 wt% in the aerosol composition. When the amount of the light liquefied gas is less than 3 wt%, an effect obtained by adding the light liquefied gas is difficult to obtain, and when the amount is more than 80 wt%, inflammability is increased.

Here, the liquid density means a value obtained by liquefying a liquefied gas in a pressure resistant cylinder and measuring a density of a liquid at 20°C with a hydrometer.

Theweight ratio (a/b) of the heavy liquefied gas (a) and the light liquefied gas (b) is preferably 5/95 to 95/5, further preferably 10/90 to 90/10, from the viewpoint of an effect of suppressing inflammability, and for making easy emulsification of the aqueous concentrate and the liquefied gas and improving emulsification stability by making a difference in a liquid density between the liquefied gas and the aqueous concentrate. A volume ratio thereof is preferably 3/97 to 97/3, further preferably 7/93 to 90/10. The difference in a liquid density between the liquefied gas and the aqueous concentrate is preferably not more than 0.35, further preferably not more than 0.3. The liquid density dmix of the liquefied gas is obtained by the following equation assuming that an amount (wt%) of the heavy liquefied gas in the liquefied gas is represented by X, a liquid density thereof is represented by $X_d$, an amount (wt%) of the light liquefied gas is represented by Y, and a liquid density thereof is represented by $Y_d$.

$$d_{mix} = (X+Y)/(X/X_d + Y/Y_d)$$

In the aerosol composition forming many independent foams in the form of soap bubble, when the heavy liquefied gas is used together with other liquefied gas, the content of the heavy liquefied gas is preferably not less than 20 wt% in the liquefied gas. When the content of the heavy liquefied gas is less than 20 wt%, there is a tendency that the foams in the form of soap bubble hardly fall and remain scattered in the air for a long period of time. The content of the heavy liquefied gas is preferably not less than 50 wt%, further preferably not less than 75 wt% in that it is easy to adjust a size of the foams in the form of soap bubble and a falling speed of the foams.

The amount of the liquefied gas is from 40 to 90 wt%, preferably from 50 to 85 wt% in the aerosol composition. When the amount of the liquefied gas is less than 40 wt%, there is a tendency that a cracking sound of the sprayed aerosol composition is small, the sprayed aerosol composition is hardly frozen, and the foams in the form of soap bubble are hardly formed. When the amount is more than 90 wt%, emulsification is difficult to proceed.

The aerosol composition of the present invention can be filled in an aerosol container, for example by filling the aqueous concentrate and the liquefied gas in a pressure resistant container, fixing an aerosol valve to the pressure resistant container, thus assembling the aerosol container, and emulsifying the aqueous concentrate and the liquefied gas. With respect to the liquefied gas, the heavy liquefied gas and the light liquefied gas are mixed previously in a specific ratio, and these gases can be filled at the same time as a liquefied gas mixture.

Moreover, compressed gas such as carbon dioxide gas, nitrogen gas, compressed air or oxygen gas can be used as a pressurizing gas for adjusting the pressure of the aerosol composition.

In aerosol products comprising the aerosol composition of the present invention, while the aqueous concentrate and the liquefied gas are emulsified in the aerosol container to form the aerosol composition, by spraying this aerosol composition from the aerosol container to the outside, the sprayed aerosol composition can be in a form of sherbet since the aqueous concentrate is cooled due to heat absorption by vaporization of the liquefied gas, can be formed into foams holding the liquefied gas therein for a long period of time and emitting a foam-cracking sound when the foams are broken, or can be formed into independent foams in the form of soap bubble.

In the case where the sprayed aerosol composition of the present invention is formed into a form of sherbet and into foams emitting a foam-cracking sound, it can be suitably used for products for human body such as a cooling agent, antiphlogistic anodynes, anti-itchings, medicine for athlete's foot, astringents, sunscreen and repellents, vermin repellent such as cooling vermicides, products for spraying on daily personal goods such as handkerchief, wet tissue, clothes, caps and hats and shoes for cooling, deodorizing and sterilizing, and other products.

Further, In the case where the sprayed aerosol composition of the present invention is sprayed to be formed into a form of soap bubble, when the aerosol composition contains a specified amount of heavy liquefied gas having a specific liquid density, a size and a falling speed of foams formed in the form of soap bubble by spraying can be adjusted. This allows suspended matters floating in a space, for example, house dusts and pollens to deposit on the foams and easily fall, thereby increasing an effect of eliminating suspended matters from a space. Further, it is possible to confirm with naked eyes that the foams in the form of soap bubble are falling in a space. Namely, by spraying the aerosol composition of the present invention in a space, the foams in the form of soap bubble can be suspended temporarily in a targeted space and the foams in the form of soap bubble fall at an adjusted falling while suspended matters such as house dusts and pollens are adhered thereto. Thereafter, according to the need, by wiping a floor, etc. on which the foams have fallen, house dusts and pollens in a space can be removed. Further, since the falling foams in the form of soap bubble can be confirmed with naked eyes, it is possible to confirm easily that all foams have fallen. Therefore there is no case of sucking the sprayed composition into a human body, which ensures safety.

The present invention is specifically explained below by means of Examples, but is not limited thereto.

Methods for evaluation are described below.

1. Number of shakes

Aerosol product is subjected to shaking 30 cm up and down, respectively, and the number of shakes until emulsification is completed is determined.

◎: Emulsification occurs by shaking 20 times or less.
○: Emulsification occurs by shaking 21 to 50 times.
△: Emulsification occurs by shaking 51 to 99 times.
✕: No emulsification occurs even by shaking 100 times.

2. Emulsification stability

Aerosol product is left at rest in a 25°C thermostatic chamber, and evaluation is made by determining a time period until separation occurs.

◎: No separation occurs for two hours or more.
○: Separation occurs within 1 to 2 hours.
△: Separation occurs within a time period of from 1 min to 1 hr.
✕: Separation occurs within one minute.
-: Not evaluated as no emulsification occurs.

3. Characteristic of product

Aerosol product is allowed to stand in a 25°C thermostatic water chamber for 30 minutes, and then conditions of a sprayed aerosol composition is evaluated.

<Cracking foam>

Evaluation is conducted by spraying a product on a person's palm.

◎: A large foam-cracking sound is produced even without rubbing a sprayed aerosol composition with palms.
○: A large foam-cracking sound is produced by rubbing a sprayed aerosol composition with palms.
△: A small foam-cracking sound is produced by rubbing a sprayed aerosol composition with palms.
✕1: No foam-cracking sound is produced even by rubbing a sprayed aerosol composition with palms.
✕2: No foam was formed.

<Sherbet spray>

Aerosol product is sprayed on an arm from a distance of 15 cm, and evaluation is carried out.

◎: The whole of sprayed aerosol composition is frozen in a form of hard sherbet and attached to an arm.
○: Eighty percent or more of the whole of sprayed aerosol composition is frozen in a form of sherbet and attached to an arm.
△: Fifty percent or less of the whole of sprayed aerosol composition is frozen in a form of sherbet and attached to an arm.
✕1: Sprayed aerosol composition is not frozen.
✕2: Sprayed aerosol composition is repelled and is not attached to an arm.

<Soap bubble spray>

a) Spray condition

Evaluation is conducted by spraying in a space.

○: Sprayed aerosol composition is formed into foams in the form of soap bubble and the foams floated in a space.
✕1: Sprayed aerosol composition is not formed into foams in the form of soap bubble, but is in the form of mist.
✕2: Sprayed aerosol composition is not formed into foams in the form of soap bubble, but is in the form of large particles.

b) Diameter of soap bubble

Aerosol product is sprayed in a horizontal direction at a height of 15 cm from a flat plate, and a diameter of foam in the form of soap bubble having fallen on the plate is measured. In Examples and Comparative Examples, diameters of five each of foams in the form of soap bubble are measured, and an average thereof is determined.

c) Falling speed of soap bubble

Aerosol product is sprayed from a height of 100 cm, and a period of time until all foams in the form of soap bubble fall on a ground just after the spraying is measured to determine a falling speed of soap bubbles.

4. Inflammability

<Cracking foam>

Aerosol product is sprayed onto a smooth base plate for one second, and is fired. A height of a flame and a firing period of time are measured
◎: Not more than 5 cm, 3 seconds or less
○: Not more than 35 cm, 10 seconds or less
Δ: Not more than 35 cm, 11 to 30 seconds
✕: Not less than 36 cm, 31 seconds or more
-: Not measurable as aerosol composition cannot be sprayed

<Sherbet spray, soap bubble spray>

Aerosol product is sprayed onto a 5 cm high flame located 15 cm apart from a spray nozzle, and elongation of the flame is measured.
◎1: No elongation is recognized.
◎2: Not more than 45 cm, and there is no back fire.
○: 46 to 50 cm (including a length of a back fire if there is the back fire)
Δ: 51 to 75 cm (including a length of a back fire if there is the back fire)
✕: 76 cm or more (including a length of a back fire if there is the back fire)

EXAMPLES 1 to 3 and COMPARATIVE EXAMPLES 1 to 3

(Cracking foam)

The following aqueous concentrate 1 was prepared, and was filled in a pressure resistant polyethylene terephthalate container in an amount shown in Table 1. An aerosol valve for inverted use was fit to the pressure resistant container, and a heavy liquefied gas (*1) and a light liquefied gas (*2) were filled as liquefied gases in amounts shown in Table 1 and Table 2. Then, the aerosol container was shaken up and down for emulsification of the aqueous concentrate and the liquefied gases to prepare an aerosol composition. The liquid density of the aqueous concentrate 1 was 0.95 g/ml. The results of evaluation are shown in Table 3.

(Aqueous concentrate 1)

| | |
|---|---|
| PEG-20 sorbitan cocoate (*3) | 0.5 |
| Talc (*4) | 0.5 |
| Ethanol | 20.0 |
| Purified water | 79.0 |

(continued)

| Total (wt%) | 100.0 |
|---|---|

*1: HFO-1234ze (liquid density at 20°C: 1.19 g/ml)
*2: Liquefied petroleum gas (liquid density at 20°C: 0.57 g/ml)
*3: NIKKOL TL10 (trade name), HLB: 16.9, available from Nikko Chemicals Co., Ltd.
*4: Crown Talc PP (trade name) available from Matsumura Sangyo Co., Ltd.

TABLE 1

| | Ex. 1 | | Ex. 2 | | Ex. 3 | |
|---|---|---|---|---|---|---|
| Aqueous concentrate 1 | 38.1 | 28.0 | 36.9 | 28.0 | 34.7 | 28.0 |
| Heavy liquefied gas | 6.1 | 3.6 | 11.9 | 7.2 | 22.4 | 14.4 |
| Light liquefied gas | 55.8 | 68.4 | 51.2 | 64.8 | 42.9 | 57.6 |
| Total | 100wt% | 100vol% | 100wt% | 100vol% | 100wt% | 100vol% |

| | Com. Ex. 1 | | Com. Ex. 2 | | Com. Ex. 3 | |
|---|---|---|---|---|---|---|
| Aqueous concentrate 1 | 39.3 | 28.0 | 7.3 | 5.0 | 64.8 | 55.0 |
| Heavy liquefied gas | - | - | 17.4 | 9.5 | 6.6 | 4.5 |
| Light liquefied gas | 60.7 | 72.0 | 75.2 | 85.5 | 28.6 | 40.5 |
| Total | 100wt% | 100vol% | 100wt% | 100vol% | 100wt% | 100vol% |

EP 2 581 431 B1

TABLE 2

|  | Ex. 1 | | Ex. 2 | | Ex. 3 | |
|---|---|---|---|---|---|---|
| Heavy liquefied gas | 9.9 | 5.0 | 18.8 | 10.0 | 34.3 | 20.0 |
| Light liquefied gas | 90.1 | 95.0 | 81.2 | 90.0 | 65.7 | 80.0 |
| Total | 100wt% | 100vol% | 100wt% | 100vol% | 100wt% | 100vol% |

|  | Com. Ex. 1 | | Com. Ex. 2 | | Com. Ex. 3 | |
|---|---|---|---|---|---|---|
| Heavy liquefied gas | - | - | 18.8 | 10.0 | 18.8 | 10.0 |
| Light liquefied gas | 100.0 | 100.0 | 81.2 | 90.0 | 81.2 | 90.0 |
| Total | 100wt% | 100vol% | 100wt% | 100vol% | 100wt% | 100vol% |

TABLE 3

|  | No. of shakes | Emulsification stability | Characteristic of Product | Inflammability |
|---|---|---|---|---|
| Ex. 1 | ○ | ○ | ○ | Δ |
| Ex. 2 | ○ | ○ | ○ | ○ |
| Ex. 3 | ◎ | ○ | Δ | ○ |
| Com. Ex. 1 | ○ | ○ | ○ | × |
| Com. Ex. 2 | × | - | ×1 | - |
| Com. Ex. 3 | × | - | ×2 | - |

EXAMPLES 4 to 8 and COMPARATIVE EXAMPLES 4 to 6

(Cracking foam)

The following aqueous concentrate 2 was prepared, and was filled in a pressure resistant polyethylene terephthalate container in an amount shown in Table 4. An aerosol valve for inverted use was fit to the pressure resistant container, and a heavy liquefied gas (*1) and a light liquefied gas (*2) were filled as liquefied gases in amounts shown in Table 4 and Table 5. Then, the aerosol container was shaken up and down for emulsification of the aqueous concentrate and the liquefied gases to prepare an aerosol composition. The liquid density of the aqueous concentrate 2 was 0.96 g/ml. The results of evaluation are shown in Table 8.

(Aqueous concentrate 2)

| | |
|---|---|
| PEG-20 sorbitan cocoate (*3) | 0.5 |
| Talc (*4) | 0.5 |
| Hydroxyethyl cellulose (*5) | 1.0 |
| Ethanol | 20.0 |
| Purified water | 78.0 |
| Total (wt%) | 100.0 |

*5: Daicel HEC-SE850 (trade name) available from Daicel Chemical Industries, Ltd.

TABLE 4

| | Ex. 4 | | Ex. 5 | | Ex. 6 | | Ex. 7 | |
|---|---|---|---|---|---|---|---|---|
| Aqueous concentrate 2 | 38.3 | 28.0 | 37.2 | 28.0 | 35.0 | 28.0 | 33.1 | 28.0 |
| Heavy liquefied gas | 6.1 | 3.6 | 11.8 | 7.2 | 22.3 | 14.4 | 31.6 | 21.6 |
| Light liquefied gas | 55.6 | 68.4 | 51.0 | 64.8 | 42.7 | 57.6 | 35.3 | 50.4 |
| Total | 100wt% | 100vol% | 100wt% | 100vol% | 100wt% | 100vol% | 100wt% | 100vol% |

| | Ex. 8 | | Com. Ex. 4 | | Com. Ex. 5 | | Com. Ex. 6 | |
|---|---|---|---|---|---|---|---|---|
| Aqueous concentrate 2 | 31.3 | 28.0 | 39.6 | 28.0 | 7.4 | 5.0 | 65.0 | 55.0 |
| Heavy liquefied gas | 40.0 | 28.8 | - | - | 17.4 | 9.5 | 6.6 | 4.5 |
| Light liquefied gas | 28.7 | 43.2 | 60.4 | 72.0 | 75.2 | 85.5 | 28.4 | 40.5 |
| Total | 100wt% | 100vol% | 100wt% | 100vol% | 100wt% | 100vol% | 100wt% | 100vol% |

TABLE 5

|  | Ex. 4 | | Ex. 5 | | Ex. 6 | | Ex. 7 | |
|---|---|---|---|---|---|---|---|---|
| Heavy liquefied gas | 9.9 | 5.0 | 18.8 | 10.0 | 34.3 | 20.0 | 47.2 | 30.0 |
| Light liquefied gas | 90.1 | 95.0 | 81.2 | 90.0 | 65.7 | 80.0 | 52.8 | 70.0 |
| Total | 100wt% | 100vol% | 100wt% | 100vol% | 100wt% | 100vol% | 100wt% | 100vol% |

|  | Ex. 8 | | Com. Ex. 4 | | Com. Ex. 5 | | Com. Ex. 6 | |
|---|---|---|---|---|---|---|---|---|
| Heavy liquefied gas | 58.2 | 40.0 | - | - | 18.8 | 10.0 | 18.8 | 10.0 |
| Light liquefied gas | 41.8 | 60.0 | 100.0 | 100.0 | 81.2 | 90.0 | 81.2 | 90.0 |
| Total | 100wt% | 100vol% | 100wt% | 100vol% | 100wt% | 100vol% | 100wt% | 100vol% |

EXAMPLES 38 to 41 and COMPARATIVE EXAMPLES 12 to 13

(Cracking foam)

The following aqueous concentrate 5 was prepared, and was filled in a pressure resistant polyethylene terephthalate container in an amount shown in Table 6. An aerosol valve for inverted use is fit to the pressure resistant container, and a heavy liquefied gas (*1) and a light liquefied gas (*2) were filled as liquefied gases in amounts shown in Table 6 and Table 7. Then, the aerosol container was shaken up and down for emulsification of the aqueous concentrate and the liquefied gases to prepare an aerosol composition. The liquid density of the aqueous concentrate 5 was 0.96 g/ml. The results of evaluation are shown in Table 8.

(Aqueous concentrate 5)

PEG-20 sorbitan cocoate (*3) 1.0

| | |
|---|---|
| Talc (*4) | 0.5 |
| Hydroxyethyl cellulose (*5) | 1.0 |
| Ethanol | 20.0 |
| Concentrated glycerin | 1.0 |
| Cyclopentasiloxane (*20) | 5.0 |
| Methylpentanediol dineopentanoate (*21) | 5.0 |
| Purified water | 66.5 |
| Total (wt%) | 100.0 |

*20: DC345 (trade name) available from Dow Corning Toray Co., Ltd.
*21: Neosolue MP (trade name) available from Nippon Fine Chemical Co., Ltd.

## TABLE 6

| | Ex. 38* | | Ex. 39* | | Ex. 40 | |
|---|---|---|---|---|---|---|
| Aqueous concentrate 5 | 20.0 | 23.6 | 30.0 | 34.7 | 40.0 | 37.7 |
| Heavy liquefied gas | 80.0 | 76.4 | 70.0 | 65.3 | 40.0 | 30.5 |
| Light liquefied gas | - | - | - | - | 20.0 | 31.8 |
| Total | 100wt% | 100vol% | 100wt% | 100vol% | 100wt% | 100vol% |

| | Ex. 41 | | Com. Ex. 12 | | Com. Ex. 13 | |
|---|---|---|---|---|---|---|
| Aqueous concentrate 5 | 20.0 | 22.5 | 5.0 | 6.1 | 65.0 | 69.7 |
| Heavy liquefied gas | 75.0 | 68.1 | 95.0 | 93.9 | 35.0 | 30.3 |
| Light liquefied gas | 5.0 | 9.4 | - | - | - | - |
| Total | 100wt% | 100vol% | 100wt% | 100vol% | 100wt% | 100vol% |

* not according to the invention

TABLE 7

| | Ex. 38* | | Ex. 39* | | Ex. 40 | |
|---|---|---|---|---|---|---|
| Heavy liquefied gas | 100.0 | 100.0 | 100.0 | 100.0 | 66.7 | 48.9 |
| Light liquefied gas | - | - | - | - | 33.3 | 51.1 |
| Total | 100wt% | 100vol% | 100wt% | 100vol% | 100wt% | 100vol% |

- continued -

* not according to the invention

- continued -

| | Ex. 41 | | Com. Ex. 12 | | Com. Ex. 13 | |
|---|---|---|---|---|---|---|
| Heavy liquefied gas | 93.8 | 87.9 | 100.0 | 100.0 | 100.0 | 100.0 |
| Light liquefied gas | 6.2 | 12.1 | - | - | - | - |
| Total | 100wt% | 100vol% | 100wt% | 100vol% | 100wt% | 100vol% |

TABLE 8

|  | No. of shakes | Emulsification stability | Characteristic of product | Inflammability |
|---|---|---|---|---|
| Ex. 4 | ○ | ○ | ◎ | △ |
| Ex. 5 | ○ | ○ | ◎ | ○ |
| Ex. 6 | ○ | ○ | ◎ | ○ |
| Ex. 7 | ○ | ○ | ○ | ○ |
| Ex. 8 | ○ | ○ | △ | ◎ |
| Ex. 38* | △ | ○ | ◎ | ◎ |
| Ex. 39* | ○ | ○ | ○ | ◎ |
| Ex. 40 | ○ | ○ | △ | ◎ |
| Ex. 41 | ○ | ○ | ◎ | ◎ |
| Com. Ex. 4 | ○ | ○ | ◎ | × |
| Com. Ex. 5 | × | - | ×2 | - |
| Com. Ex. 6 | × | - | ×1 | - |
| Com. Ex. 12 | × | - | ×2 | - |
| Com. Ex. 13 | × | - | ×1 | - |
| * not according to the invention | | | | |

EXAMPLE 9

(Cracking foam)

An aerosol composition was prepared in the same manner as in Example 5 except that polyglyceryl-10 oleate (*6) was used instead of PEG-20 sorbitan cocoate (*3). The results of evaluation are shown in Table 9.
*6: DECAGLYN 1-OV (trade name) available from Nikko Chemicals Co., Ltd.

EXAMPLE 10

(Cracking foam)

An aerosol composition was prepared in the same manner as in Example 5 except that sodium acyl glutamate (*7) was used instead of PEG-20 sorbitan cocoate (*3). The results of evaluation are shown in Table 9.
*7: AMISOFT LS11 (trade name) available from AJINOMOTO CO., INC.

EXAMPLE 11

(Cracking foam)

An aerosol composition was prepared in the same manner as in Example 5 except that triethanolamine N-cocoyl-DL-alaninate, water (*8) was used instead of PEG-20 sorbitan cocoate (*3). The results of evaluation are shown in Table 9.
*8: AMILITE ACT-12 (trade name) available from AJINOMOTO CO., INC.

EXAMPLE 12

(Cracking foam)

An aerosol composition was prepared in the same manner as in Example 5 except that PEG-12 dimethicone (*9) was used instead of PEG-20 sorbitan cocoate (*3). The results of evaluation are shown in Table 9.

*9: SH3771M (trade name) available from Dow Corning Toray Co., Ltd.

EXAMPLE 13

(Cracking foam)

An aerosol composition was prepared in the same manner as in Example 5 except that POE (40) hydrogenated castor oil (*10) was used instead of PEG-20 sorbitan cocoate (*3). The results of evaluation are shown in Table 9.
*10: NIKKOL HCO-40 (trade name) available from Nikko Chemicals Co., Ltd.

EXAMPLE 14

(Cracking foam)

An aerosol composition was prepared in the same manner as in Example 5 except that POE (20) POP (8) cetyl ether (*11) was used instead of PEG-20 sorbitan cocoate (*3). The results of evaluation are shown in Table 9.
*11: NIKKOL PBC-44 (trade name) available from Nikko Chemicals Co., Ltd.

EXAMPLE 15

(Cracking foam)

An aerosol composition was prepared in the same manner as in Example 5 except that a liquefied petroleum gas (*12) was used as a light liquefied gas instead of the liquefied petroleum gas (*2). The results of evaluation are shown in Table 9.
*12: Liquefied petroleum gas (liquid density at 20°C: 0.55 g/ml)

EXAMPLE 16

(Cracking foam)

An aerosol composition was prepared in the same manner as in Example 5 except that a gas mixture (*13) of a liquefied petroleum gas and isopentane was used as a light liquefied gas instead of the liquefied petroleum gas (*2). The results of evaluation are shown in Table 9.
*13: Weight ratio of a liquefied petroleum gas to isopentane: 65/35 (liquid density at 20°C: 0.59 g/ml)

EXAMPLE 17

(Cracking foam)

An aerosol composition was prepared in the same manner as in Example 5 except that a gas mixture (*14) of a liquefied petroleum gas and dimethyl ether was used as a light liquefied gas instead of the liquefied petroleum gas (*2). The results of evaluation are shown in Table 9.
*14: Weight ratio of a liquefied petroleum gas to dimethyl ether: 80/20 (liquid density at 20°C: 0.58 g/ml)

EXAMPLE 18

(Cracking foam)

An aerosol composition was prepared in the same manner as in Example 5 except that a gas mixture (*15) of a liquefied petroleum gas and dimethyl ether was used as a light liquefied gas instead of the liquefied petroleum gas (*2). The results of evaluation are shown in Table 9.
*15: Weight ratio of a liquefied petroleum gas to dimethyl ether: 70/30 (liquid density at 20°C: 0.60 g/ml)

EXAMPLE 19

(Cracking foam)

An aerosol composition was prepared in the same manner as in Example 5 except that 0.05 wt% of a hydrophilic silica (*16) prepared by a dry method was used as a powder instead of talc (*4) and 78.45 wt% of purified water was used. The results of evaluation are shown in Table 9.
*16: AEROSIL #200G (trade name) available from Nippon Aerosil Co., Ltd.

EXAMPLE 20

(Cracking foam)

An aerosol composition was prepared in the same manner as in Example 7 except that 0.05 wt% of a hydrophilic silica (*16) prepared by a dry method was used as a powder instead of talc (*4) and 78.45 wt% of purified water was used. The results of evaluation are shown in Table 9.

TABLE 9

|  | No. of shakes | Emulsification stability | Characteristic of Product | Inflammability |
|---|---|---|---|---|
| Ex. 9 | ○ | ○ | ◎ | ○ |
| Ex. 10 | ○ | ○ | ◎ | ○ |
| Ex 11 | ○ | ○ | ◎ | ○ |
| Ex 12 | ○ | ○ | ○ | ○ |
| Ex. 13 | ○ | ○ | Δ | ○ |
| Ex. 14 | ○ | ○ | ◎ | ○ |
| Ex. 15 | ○ | ○ | ◎ | ○ |
| Ex. 16 | ○ | ○ | ○ | Δ |
| Ex. 17 | ○ | ○ | ○ | ○ |
| Ex. 18 | Δ | Δ | Δ | ○ |
| Ex. 19 | ◎ | ◎ | ◎ | ○ |
| Ex. 20 | ◎ | ◎ | ○ | ○ |

EXAMPLES 21 to 27 and COMPARATIVE EXAMPLES 7 to 9

(Sherbet spray)

The following aqueous concentrate 3 was prepared, and was filled in a pressure resistant polyethylene terephthalate container in an amount shown in Table 10. An aerosol valve not equipped with a vapor tap orifice was fit to the pressure resistant container, and a heavy liquefied gas (*1) and a light liquefied gas (*2) were filled as liquefied gases in amounts shown in Table 10 and Table 11. Then, the aerosol container was shaken up and down for emulsification of the aqueous concentrate and the liquefied gases to prepare an aerosol composition. The liquid density of the aqueous concentrate 3 was 0.99 g/ml. The results of evaluation are shown in Table 14.

TABLE 10

| | Ex. 21 | | Ex. 22 | | Ex. 23 | | Ex. 24 | | Ex. 25 | |
|---|---|---|---|---|---|---|---|---|---|---|
| Aqueous concentrate 3 | 29.2 | 20.0 | 28.1 | 20.0 | 27.2 | 20.0 | 26.3 | 20.0 | 25.0 | 16.9 |
| Heavy liquefied gas | 7.0 | 4.0 | 13.5 | 8.0 | 19.6 | 12.0 | 25.3 | 16.0 | 8.3 | 4.7 |
| Light liquefied gas | 63.8 | 76.0 | 58.4 | 72.0 | 53.2 | 68.0 | 48.4 | 64.0 | 66.7 | 78.4 |
| Total | 100wt% | 100vol% | 100wt% | 100vol% | 100wt% | 100vol% | 100wt% | 100vol% | 100wt% | 100vol% |

| | Ex. 26 | | Ex. 27 | | Com. Ex. 7 | | Com. Ex. 8 | | Com. Ex. 9 | |
|---|---|---|---|---|---|---|---|---|---|---|
| Aqueous concentrate 3 | 20.0 | 14.2 | 40.0 | 29.6 | 30.3 | 20.0 | 7.6 | 5.0 | 65.7 | 55.0 |
| Heavy liquefied gas | 20.0 | 11.8 | 10.0 | 6.2 | - | - | 17.4 | 9.5 | 6.5 | 4.5 |
| Light liquefied gas | 60.0 | 74.0 | 50.0 | 64.3 | 69.7 | 80.0 | 75.0 | 85.5 | 27.9 | 40.5 |
| Total | 100wt% | 100vol% | 100wt% | 100vol% | 100wt% | 100vol% | 100wt% | 100vol% | 100wt% | 100vol% |

TABLE 11

| | Ex. 21 | | Ex. 22 | | Ex. 23 | | Ex. 24 | | Ex. 25 | |
|---|---|---|---|---|---|---|---|---|---|---|
| Heavy liquefied gas | 9.9 | 5.0 | 18.8 | 10.0 | 26.9 | 15.0 | 34.3 | 20.0 | 11.1 | 5.6 |
| Light liquefied gas | 90.1 | 95.0 | 81.2 | 90.0 | 73.1 | 85.0 | 65.7 | 80.0 | 88.9 | 94.4 |
| Total | 100wt% | 100vol% | 100wt% | 100vol% | 100wt% | 100vol% | 100wt% | 100vol% | 100wt% | 100vol% |

| | Ex. 26 | | Ex. 27 | | Com. Ex. 7 | | Com. Ex. 8 | | Com. Ex. 9 | |
|---|---|---|---|---|---|---|---|---|---|---|
| Heavy liquefied gas | 25.0 | 13.8 | 16.7 | 8.7 | - | - | 18.8 | 10.0 | 18.8 | 10.0 |
| Light liquefied gas | 75.0 | 86.2 | 83.3 | 91.3 | 100.0 | 100.0 | 81.2 | 90.0 | 81.2 | 90.0 |
| Total | 100wt% | 100vol% | 100wt% | 100vol% | 100wt% | 100vol% | 100wt% | 100vol% | 100wt% | 100vol% |

EP 2 581 431 B1

(Aqueous concentrate 3)

| | |
|---|---|
| POE (20) POP (8) cetyl ether (*11) | 1.0 |
| Talc (*4) | 0.5 |
| Ethanol | 5.0 |
| Purified water | 93.5 |
| Total (wt%) | 100.0 |

EXAMPLES 42 to 45 and COMPARATIVE EXAMPLES 14 to 15

(Sherbet spray)

The following aqueous concentrate 6 was prepared, and was filled in a pressure resistant polyethylene terephthalate container in an amount shown in Table 12. An aerosol valve not equipped with a vapor tap orifice was fit to the pressure resistant container, and a heavy liquefied gas (*1) and a light liquefied gas (*2) were filled as liquefied gases in amounts shown in Table 12 and Table 13. Then, the aerosol container was shaken up and down for emulsification of the aqueous concentrate and the liquefied gases to prepare an aerosol composition. The liquid density of the aqueous concentrate 6 was 0.99 g/ml. The results of evaluation are shown in Table 14.

(Aqueous concentrate 6)

| | |
|---|---|
| POE (20) POP (8) cetyl ether (*11) | 1.0 |
| Talc (*4) | 0.5 |
| Ethanol | 2.0 |
| Purified water | 96.5 |
| Total (wt%) | 100.0 |

TABLE 12

| | Ex. 42* | | Ex. 43* | | Ex. 44 | |
|---|---|---|---|---|---|---|
| Aqueous concentrate 6 | 13.8 | 16.1 | 25.0 | 28.7 | 30.0 | 26.0 |
| Heavy liquefied gas | 86.2 | 83.9 | 75.0 | 71.3 | 40.0 | 28.8 |
| Light liquefied gas | - | - | - | - | 30.0 | 45.2 |
| Total | 100wt% | 100vol% | 100wt% | 100vol% | 100wt% | 100vol% |

- continued -

| | Ex. 45 | | Com. Ex. 14 | | Com. Ex. 15 | |
|---|---|---|---|---|---|---|
| Aqueous concentrate 6 | 15.0 | 15.9 | 5.0 | 6.0 | 65.0 | 69.1 |
| Heavy liquefied gas | 75.0 | 65.8 | 95.0 | 94.0 | 35.0 | 30.9 |
| Light liquefied gas | 10.0 | 18.3 | - | - | - | - |
| Total | 100wt% | 100vol% | 100wt% | 100vol% | 100wt% | 100vol% |

- continued -

* not according to the invention

26

TABLE 13

| | Ex. 42* | | Ex. 43* | | Ex. 44 | |
|---|---|---|---|---|---|---|
| Heavy liquefied gas | 100.0 | 100.0 | 100.0 | 100.0 | 57.1 | 39.1 |
| Light liquefied gas | - | - | - | - | 42.9 | 60.9 |
| Total | 100wt% | 100vol% | 100wt% | 100vol% | 100wt% | 100vol% |

- continued -

- continued -

| | Ex. 45 | | Com. Ex. 14 | | Com. Ex. 15 | |
|---|---|---|---|---|---|---|
| Heavy liquefied gas | 88.2 | 78.2 | 100.0 | 100.0 | 100.0 | 100.0 |
| Light liquefied gas | 11.8 | 21.8 | - | - | - | - |
| Total | 100wt% | 100vol% | 100wt% | 100vol% | 100wt% | 100vol% |

* not according to the invention

TABLE 14

|  | No. of shakes | Emulsification stability | characteristic of product | Inflammability |
|---|---|---|---|---|
| Ex. 21 | ◎ | ○ | ◎ | Δ |
| Ex. 22 | ◎ | ○ | ◎ | ○ |
| Ex. 23 | ◎ | ○ | ◎ | ○ |
| Ex. 24 | ◎ | ○ | ○ | ◎2 |
| Ex. 25 | ◎ | ○ | ◎ | Δ |
| Ex. 26 | ◎ | ○ | ○ | ◎2 |
| Ex. 27 | ○ | ○ | Δ | ◎2 |
| Ex. 42* | Δ | ○ | ◎ | ◎1 |
| Ex. 43* | ○ | ○ | ○ | ◎1 |
| Ex. 44 | ○ | ○ | Δ | ◎1 |
| Ex. 45 | ○ | ○ | ◎ | ◎1 |
| Com. Ex. 7 | ◎ | ○ | ◎ | × |
| Com. Ex. 8 | × | - | ×2 | - |
| Com. Ex. 9 | × | - | ×1 | - |
| Com. Ex. 14 | × | - | ×2 | - |
| Com. Ex. 15 | × | - | ×1 | - |
| * not according to the invention | | | | |

EXAMPLE 28

(Sherbet spray)

An aerosol composition was prepared in the same manner as in Example 23 except that triethanolamine N-cocoyl-DL-alaninate, water (*8) was used instead of POE (20) POP (8) cetyl ether (*11). The results of evaluation are shown in Table 15.

EXAMPLE 29

(Sherbet spray)

An aerosol composition was prepared in the same manner as in Example 23 except that POE (40) hydrogenated castor oil (*10) was used instead of POE (20)POP(8) cetyl ether (*11). The results of evaluation are shown in Table 15.

EXAMPLE 30

(Sherbet spray)

An aerosol composition was prepared in the same manner as in Example 23 except that a liquefied petroleum gas (*12) was used as a light liquefied gas instead of the liquefied petroleum gas (*2). The results of evaluation are shown in Table 15.

EXAMPLE 31

(Sherbet spray)

An aerosol composition was prepared in the same manner as in Example 23 except that a gas mixture (*13) of a liquefied petroleum gas and isopentane was used as a light liquefied gas instead of the liquefied petroleum gas (*2). The results of evaluation are shown in Table 15.

EXAMPLE 32

(Sherbet spray)

An aerosol composition was prepared in the same manner as in Example 23 except that a gas mixture (*14) of a liquefied petroleum gas and dimethyl ether was used as a light liquefied gas instead of the liquefied petroleum gas (*2). The results of evaluation are shown in Table 15.

EXAMPLE 33

(Sherbet spray)

An aerosol composition was prepared in the same manner as in Example 23 except that a gas mixture (*15) of a liquefied petroleum gas and dimethyl ether was used as a light liquefied gas instead of the liquefied petroleum gas (*2). The results of evaluation are shown in Table 15.

EXAMPLE 34

(Sherbet spray)

An aerosol composition was prepared in the same manner as in Example 22 except that 0.05 wt% of a hydrophilic silica (*16) prepared by a dry method was used as a powder instead of talc (*4) and 78.45 wt% of purified water was used. The results of evaluation are shown in Table 15.

EXAMPLE 35

(Sherbet spray)

An aerosol composition was prepared in the same manner as in Example 24 except that 0.05 wt% of a hydrophilic silica (*16) prepared by a dry method was used as a powder instead of talc (*4) and 78.45 wt% of purified water was used. The results of evaluation are shown in Table 15.

TABLE 15

|  | No. of shakes | Emulsification stability | Characteristic of product | Inflammability |
|---|---|---|---|---|
| Ex. 28 | ◎ | ○ | ◎ | ○ |
| Ex. 29 | ◎ | ○ | ◎ | ○ |
| Ex. 30 | ◎ | ○ | ◎ | ○ |
| Ex. 31 | ◎ | ○ | ○ | Δ |
| Ex. 32 | ◎ | ○ | ◎ | ○ |
| Ex. 33 | ○ | ○ | ○ | ○ |
| Ex. 34 | ◎ | ◎ | ◎ | ○ |
| Ex. 35 | ◎ | ◎ | ○ | ○ |

EXAMPLES 36 and 37 and COMPARATIVE EXAMPLES 10 and 11

(Soap bubble spray)

The following aqueous concentrate 4 was prepared, and was filled in a pressure resistant polyethylene terephthalate container in an amount shown in Table 16. An aerosol valve not equipped with a vapor tap orifice was fit to the pressure resistant container, and a heavy liquefied gas (*1) and a light liquefied gas (*2) were filled as liquefied gases

EP 2 581 431 B1

in amounts shown in Table 16 and Table 17. Then, the aerosol container was shaken up and down for emulsification of the aqueous concentrate and the liquefied gases to prepare an aerosol composition. The liquid density of the aqueous concentrate 4 was 0.93 g/ml. The results of evaluation are shown in Table 20.

(Aqueous concentrate 4)

| POE (30) cetyl ether (*17) | 2.0 |
| Stearyl trimethyl ammonium chloride (*18) | 2.0 |
| Methyl polysiloxane (*19) | 2.0 |
| Methylparaben | 0.1 |
| Ethanol | 30.0 |
| Purified water | 63.9 |
| Total (wt%) | 100.0 |

*17: BC-30TX (trade name) available from Nikko Chemicals Co., Ltd.
*18: QUARTAMIN 86PC (trade name) available from KAO CORPORATION
*19: SH200 5cs (trade name) available from Dow Corning Toray Co., Ltd.

TABLE 16

| | Ex. 36 | | Ex. 37 | | Com. Ex. 10 | | Com. Ex. 11 | |
|---|---|---|---|---|---|---|---|---|
| Aqueous concentrate 4 | 26.9 | 20.0 | 25.1 | 20.0 | 7.2 | 5.0 | 64.3 | 55.0 |
| Heavy liquefied gas | 13.8 | 8.0 | 25.7 | 16.0 | 17.5 | 9.5 | 6.7 | 4.5 |
| Light liquefied gas | 59.3 | 72.0 | 49.2 | 64.0 | 75.3 | 85.5 | 29.0 | 40.5 |
| Total | 100wt% | 100vol% | 100wt% | 100vol% | 100wt% | 100vol% | 100wt% | 100vol% |

TABLE 17

| | Ex. 36 | | Ex. 37 | | Com. Ex. 10 | | Com. Ex. 11 | |
|---|---|---|---|---|---|---|---|---|
| Heavy liquefied gas | 18.8 | 10.0 | 34.3 | 20.0 | 18.8 | 10.0 | 18.8 | 10.0 |
| Light liquefied gas | 81.2 | 90.0 | 65.7 | 80.0 | 81.2 | 90.0 | 81.2 | 90.0 |
| Total | 100wt% | 100vol% | 100wt% | 100vol% | 100wt% | 100vol% | 100wt% | 100vol% |

EXAMPLES 46 to 49 and COMPARATIVE EXAMPLES 16 to 18 (Soap bubble spray)

The following aqueous concentrate 7 was prepared, and was filled in a pressure resistant polyethylene terephthalate container in an amount shown in Table 18. An aerosol valve not equipped with a vapor tap orifice was fit to the pressure resistant container, and a heavy liquefied gas (*1) and a light liquefied gas (*2) were filled as liquefied gases in amounts shown in Table 18 and Table 19. Then, the aerosol container was shaken up and down for emulsification of the aqueous concentrate and the liquefied gases to prepare an aerosol composition. The liquid density of the aqueous concentrate 7 was 0.96 g/ml. The results of evaluation are shown in Table 20.

(Aqueous concentrate 7)

| POE (20) cetyl ether (*22) | 3.0 |
| Sodium N-cocoyl-L-glutamate (*23) | 2.0 |
| Polyoxyethylene/methyl polysiloxane copolymer (*7) | 3.0 |

(continued)

| | |
|---|---|
| Aqueous solution of sodium carboxymethyl cellulose (*24) | 20.0 |
| Silica (*16) | 0.1 |
| Talc (*4) | 0.3 |
| Concentrated glycerin | 10.0 |
| Ethanol | 21.0 |
| Purified water | 40.6 |
| Total (wt%) | 100.0 |

*22: BC-20TX (trade name) available from Nikko Chemicals Co., Ltd.
*23: Aminosurfact ACDS-L (trade name) available from ASAHI KASEI CHEMICALS CORPORATION
*24: 0.5% aqueous solution of Cellogen P-815C (trade name) available from DAI-ICHI KOGYO SEIYAKU CO., LTD.

EP 2 581 431 B1

TABLE 18

|  | Ex. 46* | | Ex. 47* | | Ex. 48* | | Ex. 49 | |
|---|---|---|---|---|---|---|---|---|
| Aqueous concentrate 7 | 50.0 | 55.4 | 40.0 | 45.3 | 30.0 | 34.7 | 30.0 | 31.6 |
| Heavy liquefied gas | 50.0 | 44.6 | 60.0 | 54.7 | 70.0 | 65.3 | 60.0 | 50.8 |
| Light liquefied gas | - | - | - | - | - | - | 10.0 | 17.6 |
| Total | 100wt% | 100vol% | 100wt% | 100vol% | 100wt% | 100vol% | 100wt% | 100vol% |

- continued -

|  | Com. Ex. 16 | | Com. Ex. 17 | | Com. Ex. 18 | |
|---|---|---|---|---|---|---|
| Aqueous concentrate 7 | 30.0 | 20.4 | 5.0 | 6.1 | 65.0 | 69.7 |
| Heavy liquefied gas | - | - | 95.0 | 93.9 | 35.0 | 30.3 |
| Light liquefied gas | 70.0 | 79.6 | - | - | - | - |
| Total | 100wt% | 100vol% | 100wt% | 100vol% | 100wt% | 100vol% |

* not according to the invention

TABLE 19

| | Ex. 46* | | Ex. 47* | | Ex. 48* | | Ex. 49 | |
|---|---|---|---|---|---|---|---|---|
| Heavy liquefied gas | 100.0 | 100.0 | 100.0 | 100.0 | 100.0 | 100.0 | 85.7 | 74.3 |
| Light liquefied gas | - | - | - | - | | | 14.2 | 25.7 |
| Total | 100wt% | 100vol% | 100wt% | 100vol% | 100wt% | 100vol% | 100wt% | 100vol% |

| | Com. Ex. 16 | | Com. Ex. 17 | | Com. Ex. 18 | |
|---|---|---|---|---|---|---|
| Heavy liquefied gas | - | - | 100.0 | 100.0 | 100.0 | 100.0 |
| Light liquefied gas | 100.0 | 100.0 | - | - | - | - |
| Total | 100wt% | 100vol% | 100wt% | 100vol% | 100wt% | 100vol% |

* not according to the invention

TABLE 20

|  | No. of shakes | Emulsification stability | characteristic of product | Diameter of soap bubble (mm) | Falling speed of soap bubble (m/sec) | Inflammability |
|---|---|---|---|---|---|---|
| Ex. 36 | ○ | ○ | ○ | 0.3 | 0.016 | ○ |
| Ex. 37 | ○ | ○ | ○ | 0.3 | 0.017 | ○ |
| Ex. 46* | ◎ | ◎ | ○ | 0.6 | 0.055 | ◎1 |
| Ex. 47* | ◎ | ◎ | ○ | 0.5 | 0.048 | ◎1 |
| Ex. 48* | ○ | ○ | ○ | 0.4 | 0.033 | ◎1 |
| Ex. 49 | ◎ | ○ | ○ | 0.4 | 0.029 | ◎1 |
| Com. Ex. 10 | × | - | ×1 | - | - | - |
| Com. Ex. 11 | × | - | ×2 | - | - | - |
| Com. Ex. 16 | ◎ | - | ○ | 0.2 | 0.013 | × |
| Com. Ex. 17 | × | - | ×1 | - | - | - |
| Com. Ex. 18 | × | - | ×2 | - | - | - |
| * not according to the invention | | | | | | |

EXAMPLES 50 to 58 and COMPARATIVE EXAMPLES 19 to 23 (Soap bubble spray)

The following aqueous concentrate 8 was prepared, and was filled in a pressure resistant polyethylene terephthalate container in an amount shown in Tables 21 to 23. An aerosol valve was fit to the pressure resistant container, and liquefied gases were filled in amounts shown in Tables 21 to 23. Then, the aerosol container was shaken up and down for emulsification of the aqueous concentrate and the liquefied gases to prepare an aerosol composition. The liquid density at 20°C of the aqueous concentrate 8 was 0.98 g/ml and the liquid viscosity at 20°C was 30 mPa·s. The results of evaluation are shown in Table 25.

(Aqueous concentrate 8)

| | |
|---|---|
| POE (20) cetyl ether (*22) | 3.0 |
| Sodium N-cocoyl-L-glutamate (*25) | 0.5 |
| Polyoxyethylene/methyl polysiloxane copolymer (*9) | 3.0 |
| Aqueous solution of sodium carboxymethyl cellulose (*24) | 20.0 |
| Para-hydroxybenzoic ester (*26) | 0.1 |
| Silica (*16) | 0.1 |
| Talc (*4) | 0.3 |
| Glycerin | 10.0 |
| Ethanol | 20.0 |
| Purified water | 43.0 |
| Total (wt%) | 100.0 |

*25: Amisoft CS-11 (trade name) available from AJINOMOTO CO., INC.
*26: Mekkins M (trade name) available from Ueno Fine Chemicals Industry, Ltd.

TABLE 21

| | Ex. 50* | | Ex. 51* | | Ex. 52* | | Ex. 53* | |
|---|---|---|---|---|---|---|---|---|
| Aqueous concentrate 8 | 15.0 | 17.6 | 30.0 | 34.3 | 40.0 | 44.8 | 60.0 | 64.6 |
| Liquefied gas HFO-1234ze (*1) | 85.0 | 82.4 | 70.0 | 65.7 | 60.0 | 55.2 | 40.0 | 35.4 |
| Total | 100wt% | 100vol% | 100wt% | 100vol% | 100wt% | 100vol% | 100wt% | 100vol% |

TABLE 22

| | Ex. 54* | | Ex. 55* | | Ex. 56* | | Ex. 57* | | Ex. 58* | |
|---|---|---|---|---|---|---|---|---|---|---|
| Aqueous concentrate 8 | 40.0 | 30.2 | 40.0 | 33.9 | 40.0 | 35.6 | 40.0 | 38.6 | 40.0 | 28.1 |
| Liquefied gas<br>  HFO-1234ze (*1)<br>  LPG (*27) | 15.0<br>45.0 | 9.3<br>60.5 | 30.0<br>30.0 | 20.9<br>45.2 | 36.0<br>24.0 | 26.3<br>38.1 | 45.0<br>15.0 | 35.6<br>25.8 | 5.0<br>55.0 | 2.9<br>69.0 |
| Total | 100wt% | 100vol% | 100wt%, | 100vol% | 100wt% | 100vol% | 100wt% | 100vol% | 100wt% | 100vol% |

*not according to the invention
*27: Liquefied petroleum gas (Pressure at 25°C: 0.39 MPa, Liquid density at 20°C: 0.55 g/ml)

TABLE 23

| | Com. Ex. 19 | | Com. Ex. 20 | | Com. Ex. 21 | | Com. Ex. 22 | | Com. Ex. 23 | |
|---|---|---|---|---|---|---|---|---|---|---|
| Aqueous concentrate 8 | 15.0 | 9.0 | 25.0 | 15.8 | 40.0 | 27.2 | 50.0 | 35.9 | 5.0 | 6.0 |
| Liquefied gas | | | | | | | | | | |
| HFO-1234ze (*1) | - | - | - | - | - | - | - | - | 95.0 | 94.0 |
| LPG (*27) | 85.0 | 91.0 | 75.0 | 84.2 | 60.0 | 72.8 | 50.0 | 64.1 | - | - |
| Total | 100wt% | 100vol% | 100wt% | 100vol% | 100wt% | 100vol% | 100wt% | 100vol% | 100wt% | 100vol% |

EXAMPLE 59

(Soap bubble spray)

An aerosol composition was prepared in the same manner as in Example 50 except that liquefied gases were filled in amounts shown in Table 24. The results of evaluation are shown in Table 25.

EXAMPLE 60

(Soap bubble spray)

The following aqueous concentrate 9 was prepared, and was filled in a pressure resistant polyethylene terephthalate container in an amount shown in Table 24. An aerosol valve was fit to the pressure resistant container, and liquefied gases were filled in amounts shown in Table 24. Then, the aerosol container was shaken up and down for emulsification of the aqueous concentrate and the liquefied gases to prepare an aerosol composition. The liquid density at 20°C of the aqueous concentrate 9 was 0.98 g/ml and the liquid viscosity at 20°C was 30 mPa·s. The results of evaluation are shown in Table 25.

(Aqueous concentrate 9)

| | |
|---|---|
| POE (20) cetyl ether (*22) | 6.0 |
| Sodium N-cocoyl-L-glutamate (*25) | 2.0 |
| Polyoxyethylene/methyl polysiloxane copolymer (*9) | 3.0 |
| Aqueous solution of sodium carboxymethyl cellulose (*24) | 20.0 |
| Concentrated glycerin | 10.0 |
| Ethanol (95%) | 31.0 |
| Purified water | 28.0 |
| Total (wt%) | 100.0 |

EXAMPLE 61

(Soap bubble spray)

The following aqueous concentrate 10 was prepared, and was filled in a pressure resistant polyethylene terephthalate container in an amount shown in Table 24. An aerosol valve was fit to the pressure resistant container, and liquefied gases were filled in amounts shown in Table 24. Then, the aerosol container was shaken up and down for emulsification of the aqueous concentrate and the liquefied gases to prepare an aerosol composition. The liquid density at 20°C of the aqueous concentrate 10 was 0.98 g/ml and the liquid viscosity at 20°C was 30 mPa·s. The results of evaluation are shown in Table 25.

(Aqueous concentrate 10)

| | |
|---|---|
| POE (20) cetyl ether (*22) | 5.0 |
| Sodium N-cocoyl-L-glutamate (*25) | 1.5 |
| Polyoxyethylene/methyl polysiloxane copolymer (*9) | 2.5 |
| Aqueous solution of sodium carboxymethyl cellulose (*24) | 20.0 |
| Silica (*16) | 0.1 |
| Talc (*4) | 0.3 |
| Liquid paraffin (*28) | 13.0 |
| Concentrated glycerin | 9.0 |
| Ethanol (95%) | 18.0 |

(continued)

| | |
|---|---|
| Purified water | 30.6 |
| Total (wt%) | 100.0 |
| *28: HICALL K-230 (trade name) available from KANEDA CO., LTD. | |

EXAMPLE 62

(Soap bubble spray)

The following aqueous concentrate 11 was prepared, and was filled in a pressure resistant polyethylene terephthalate container in an amount shown in Table 24. An aerosol valve was fit to the pressure resistant container, and liquefied gases were filled in amounts shown in Table 24. Then, the aerosol container was shaken up and down for emulsification of the aqueous concentrate and the liquefied gases to prepare an aerosol composition. The liquid density at 20°C of the aqueous concentrate 11 was 0.98 g/ml and the liquid viscosity at 20°C was 30 mPa·s. The results of evaluation are shown in Table 25.

(Aqueous concentrate 11)

| | |
|---|---|
| POE (20) cetyl ether (*22) | 5.0 |
| Potassium N-cocoyl glycinate (*29) | 1.5 |
| Polyoxyethylene/methyl polysiloxane copolymer (*9) | 2.5 |
| Aqueous solution of sodium carboxymethyl cellulose (*24) | 20.0 |
| Silica (*16) | 0.1 |
| Talc (*4) | 0.3 |
| Liquid paraffin (*28) | 13.0 |
| Concentrated glycerin | 9.0 |
| Ethanol (95%) | 18.0 |
| Purified water | 30.6 |
| Total (wt%) | 100.0 |
| *29: AMILITE GCK-11 (trade name) available from AJINOMOTO CO., INC. | |

TABLE 24

| | Ex. 59 | | Ex. 60* | | Ex. 61* | | Ex. 62* | |
|---|---|---|---|---|---|---|---|---|
| Aqueous concentrate 8 | 40.0 | 35.7 | - | - | - | - | - | - |
| Aqueous concentrate 9 | - | - | 25.0 | 28.9 | - | - | - | - |
| Aqueous concentrate 10 | - | - | - | - | 55.0 | 59.8 | - | - |
| Aqueous concentrate 11 | - | - | - | - | - | - | 40.0 | 44.8 |
| Liquefied gas | | | | | | | | |
| HFO-1234ze (*1) | 30.0 | 22.0 | 75.0 | 71.1 | 45.0 | 40.2 | 60.0 | 55.2 |
| Isopentane | 30.0 | 42.3 | - | - | - | - | - | - |
| Total | 100wt% | 100vol% | 100wt% | 100vol% | 100wt% | 100vol% | 100wt% | 100vol% |

TABLE 25

| | No. of shakes | Emulsification stability | Characteristic of product | Inflammability | Diameter of soap bubble (mm) | Falling speed of soap bubble (m/sec) |
|---|---|---|---|---|---|---|
| Ex. 50 | ○ | ○ | ○ | ◎1 | 0.2 | 0.028 |
| Ex. 51 | ○ | ○ | ○ | ◎1 | 0.4 | 0.033 |
| Ex. 52 | ◎ | ◎ | ○ | ◎1 | 0.5 | 0.048 |
| Ex. 53 | ◎ | ◎ | ○ | ◎1 | 0.8 | 0.125 |
| Ex. 54 | ◎ | ◎ | ○ | ○ | 0.3 | 0.016 |
| Ex. 55 | ◎ | ◎ | ○ | ○ | 0.4 | 0.020 |
| Ex. 56 | ◎ | ◎ | ○ | ○ | 0.4 | 0.026 |
| Ex. 57 | ◎ | ◎ | ○ | ◎2 | 0.5 | 0.032 |
| Ex. 58 | ◎ | ◎ | ○ | Δ | 0.3 | 0.013 |
| Ex. 59 | ◎ | ◎ | ○ | ○ | 0.8 | 0.065 |
| Ex. 60* | ◎ | ○ | ○ | ◎1 | 0.4 | 0.045 |
| Ex. 61* | ◎ | ◎ | ○ | ◎1 | 0.6 | 0.052 |
| Ex. 62* | ◎ | ◎ | ○ | ◎1 | 0.5 | 0.044 |
| Com. Ex. 19 | ◎ | ○ | ○ | × | 0.2 | 0.010 |
| Com. Ex. 20 | ◎ | ○ | ○ | × | 0.2 | 0.012 |
| Com. Ex. 21 | ◎ | ◎ | ○ | × | 0.3 | 0.013 |
| Com. Ex. 22 | ◎ | ◎ | ○ | × | 1.0 | 0.030 |
| Com. Ex. 23 | × | - | ×1 | ◎1 | - | - |

PRODUCT EXAMPLE 1

(Space treating agent)

60 grams (60 wt%) of the following aqueous concentrate was filled in an aluminum aerosol container. An aerosol valve not equipped with a vapor tap orifice was fit to the aerosol container, and 40 grams (40 wt%) of trans-1,3,3,3-tetrafluoroprop-1-ene was filled as a liquefied gas. Then, the aerosol container was shaken up and down for emulsification of the aqueous concentrate and the liquefied gas to prepare an aerosol composition. When this aerosol composition was sprayed into a space, foams in the form of soap bubble floated temporarily, and house dusts were attached to the foams and then the foams fell on a ground.

| | |
|---|---|
| POE (20) cetyl ether (*22) | 3.0 |
| Sodium N-cocoyl-L-glutamate (*25) | 0.5 |
| Polyoxyethylene/methyl polysiloxane copolymer (*9) | 3.0 |
| Aqueous solution of sodium carboxymethyl cellulose (*24) | 20.0 |
| Copolymer of ethyl acrylate/alkyl methacrylate/diacetone acrylamide/methacrylic acid (*30) | 1.0 |
| Para-hydroxybenzoic ester (*26) | 0.1 |
| Silica (*16) | 0.1 |
| Talc (*4) | 0.3 |
| 1,3-Butylene glycol | 5.0 |

(continued)

| | |
|---|---|
| Ethanol (95%) | 23.0 |
| Purified water | 40.0 |
| Total (wt%) | 100.0 |

*30: PLAS CIZE L-53 (trade name) available from GOO CHEMICAL CO., LTD.

PRODUCT EXAMPLE 2

(Deodorant, aromatic substance)

40 grams (40 wt%) of the following aqueous concentrate was filled in an aluminum aerosol container. An aerosol valve not equipped with a vapor tap orifice was fit to the aerosol container, and 12 grams (12 wt%) of a liquefied petroleum gas and 48 grams (48 wt%) of trans-1,3,3,3-tetrafluoroprop-1-ene were filled as liquefied gases. Then, the aerosol container was shaken up and down for emulsification of the aqueous concentrate and the liquefied gases to prepare an aerosol composition. When this aerosol composition was sprayed into a space, foams in the form of soap bubble floated temporarily, removed odor in a space and then fell on a ground.

| | |
|---|---|
| POE (20) cetyl ether (*22) | 3.0 |
| Sodium lauroyl sarcosinate (*31) | 1.0 |
| Polyoxyethylene/methyl polysiloxane copolymer (*9) | 3.0 |
| Aqueous solution of sodium carboxymethyl cellulose (*24) | 20.0 |
| Hydroxyethyl acrylate/methoxyethyl acrylate copolymer (*32) | 3.0 |
| Para-hydroxybenzoic ester (*26) | 0.1 |
| Silica (*16) | 0.1 |
| Talc (*4) | 0.3 |
| Perfume | 0.1 |
| Ethanol (95%) | 15.0 |
| Purified water | 54.4 |
| Total (wt%) | 100.0 |

*31: SARCOSINATE LN (trade name) available from Nikko Chemicals Co., Ltd.
*32: PLAS CIZE L-222 (trade name) available from GOO CHEMICAL CO., LTD.

PRODUCT EXAMPLE 3

(Vermin repellents for use in a space)

60 grams (60 wt%) of the following aqueous concentrate was filled in an aluminum aerosol container. An aerosol valve not equipped with a vapor tap orifice was fit to the aerosol container, and 4 grams (4 wt%) of a liquefied petroleum gas and 36 grams (36 wt%) of trans-1,3,3,3-tetrafluoroprop-1-ene were filled as liquefied gases. Then, the aerosol container was shaken up and down for emulsification of the aqueous concentrate and the liquefied gases to prepare an aerosol composition. When this aerosol composition was sprayed into a space, foams in the form of soap bubble floated temporarily, and odor of herb extract which was a repelling component floated.

| | |
|---|---|
| POE (20) cetyl ether (*22) | 3.0 |
| Sodium N-cocoyl-L-glutamate (*25) | 1.0 |
| Polyoxyethylene/methyl polysiloxane copolymer (*9) | 2.0 |
| Aqueous solution of sodium carboxymethyl cellulose (*24) | 20.0 |
| Para-hydroxybenzoic ester (*26) | 0.1 |
| Silica (*16) | 0.1 |
| Talc (*4) | 0.3 |
| Herb extract | 2.0 |
| Ethanol (95%) | 20.0 |
| Purified water | 51.5 |

(continued)

| | |
|---|---|
| Total (wt%) | 100.0 |

## PRODUCT EXAMPLE 4

(Insecticide for use in a space)

60 grams (60 wt%) of the following aqueous concentrate was filled in an aluminum aerosol container. An aerosol valve not equipped with a vapor tap orifice was fit to the aerosol container, and 40 grams (40 wt%) of trans-1,3,3,3-tetrafluoroprop-1-ene was filled as a liquefied gas. Then, the aerosol container was shaken up and down for emulsification of the aqueous concentrate and the liquefied gas to prepare an aerosol composition. When this aerosol composition was sprayed onto a flying noxious insect, foams in the form of soap bubble floated temporarily, and were attached to the foams and then dropped.

| | |
|---|---|
| POE (20) cetyl ether (*22) | 3.0 |
| Sodium N-cocoyl-L-glutamate (*25) | 1.0 |
| Polyoxyethylene/methyl polysiloxane copolymer (*9) | 3.0 |
| Aqueous solution of sodium carboxymethyl cellulose (*24) | 20.0 |
| Para-hydroxybenzoic ester (*26) | 0.1 |
| Silica (*16) | 0.1 |
| Talc (*4) | 0.3 |
| Phthalthrin | 0.5 |
| Ethanol (95%) | 20.0 |
| Purified water | 52.0 |
| Total (wt%) | 100.0 |

## PRODUCT EXAMPLE 5

(Refrigerant)

30 grams (30 wt%) of the following aqueous concentrate was filled in an aluminum aerosol container. An aerosol valve for inverted use was fit to the aerosol container, and 60 grams (60 wt%) of trans-1,3,3,3-tetrafluoroprop-1-ene and 10 grams (10 wt%) of a liquefied petroleum gas were filled as liquefied gases. Then, the aerosol container was shaken up and down for emulsification of the aqueous concentrate and the liquefied gases to prepare an aerosol composition. When this aerosol composition was sprayed onto a palm, foams in the form of gel were formed. When these foams were smeared on an arm, a foam-cracking sound was emitted, and the arm could be cooled while being massaged.

| | |
|---|---|
| PEG-20 sorbitan cocoate (*3) | 0.5 |
| Talc (*4) | 0.5 |
| Hydroxyethyl cellulose (*5) | 0.5 |
| Ethanol | 20.0 |
| Concentrated glycerin | 1.0 |
| 1-Menthol | 0.5 |
| Cyclopentasiloxane (*20) | 5.0 |
| Methylpentanediol dineopentanoate (*21) | 5.0 |
| Perfume | 0.1 |
| Purified water | 66.9 |
| Total (wt%) | 100.0 |

PRODUCT EXAMPLE 6

(Vermin repellents)

30 grams (30 wt%) of the following aqueous concentrate was filled in an aluminum aerosol container. An aerosol valve for inverted use was fit to the aerosol container, and 50 grams (50 wt%) of trans-1,3,3,3-tetrafluoroprop-1-ene and 20 grams (20 wt%) of a liquefied petroleum gas were filled as liquefied gases. Then, the aerosol container was shaken up and down for emulsification of the aqueous concentrate and the liquefied gases to prepare an aerosol composition. When this aerosol composition was sprayed onto a palm, foams in the form of gel were formed. When these foams were smeared on an arm, a foam-cracking sound was emitted, and the arm could be coated with the aerosol composition while being massaged.

| | |
|---|---|
| PEG-20 sorbitan cocoate (*3) | 0.5 |
| Talc (*4) | 0.5 |
| Hydroxyethyl cellulose (*5) | 0.5 |
| Ethanol | 25.0 |
| 1,3-Butylene glycol | 3.0 |
| 1-Menthol | 0.5 |
| N,N-Diethyl-m-toluamide | 1.0 |
| Cyclopentasiloxane (*20) | 5.0 |
| Methylpentanediol dineopentanoate (*21) | 5.0 |
| Herb extract | 0.2 |
| Purified water | 58.8 |
| Total (wt%) | 100.0 |

PRODUCT EXAMPLE 7

(Vermin repellents)

30 grams (25 wt%) of the following aqueous concentrate was filled in an aluminum aerosol container. An aerosol valve not equipped with a vapor tap orifice was fit to the aerosol container, and 20 grams (16.7 wt%) of trans-1,3,3,3-tetrafluoroprop-1-ene and 70 grams (58.3 wt%) of a liquefied petroleum gas were filled as liquefied gases. Then, the aerosol container was shaken up and down for emulsification of the aqueous concentrate and the liquefied gases to prepare an aerosol composition. When this aerosol composition was sprayed onto a cockroach one meter apart from the container, the sprayed aerosol composition was frozen on the cockroach, and a motion of the cockroach became dull.

| | |
|---|---|
| POE (20) POP (8) cetyl ether (*11) | 1.0 |
| Talc (*4) | 0.5 |
| Ethanol | 2.0 |
| Purified water | 96.5 |
| Total (wt%) | 100.0 |

PRODUCT EXAMPLE 8

(Coolant)

30 grams (30 wt%) of the following aqueous concentrate was filled in an aluminum aerosol container. An aerosol valve not equipped with a vapor tap orifice was fit to the aerosol container, and 60 grams (60 wt%) of trans-1,3,3,3-tetrafluoroprop-1-ene and 10 grams (10 wt%) of a liquefied petroleum gas were filled as liquefied gases. Then, the aerosol container was shaken up and down for emulsification of the aqueous concentrate and the liquefied gases to prepare an aerosol composition. When this aerosol composition was sprayed onto a handkerchief, the sprayed aerosol composition was frozen, and the scuff of the neck could be cooled by placing the handkerchief thereon.

| | |
|---|---|
| POE (20) POP (8) cetyl ether (*11) | 1.0 |
| Talc (*4) | 0.5 |

(continued)

| | |
|---|---|
| 1-Menthol | 0.5 |
| Ethanol | 5.0 |
| Purified water | 93.0 |
| Total (wt%) | 100.0 |

**Claims**

1. An aerosol composition comprising:

   10 to 60 wt% of an aqueous concentrate comprising a surfactant and water and
   40 to 90 wt% of a liquefied gas, and

   obtained by emulsifying the aqueous concentrate and the liquefied gas, wherein a content of water in the aqueous concentrate is 40 to 99 wt%,
   wherein the liquefied gas comprises a heavy liquefied gas (a) having a liquid density at 20°C of from 1.15 to 1.30 (g/ml) and a light liquefied gas (b) having a liquid density at 20 °C of from 0.50 to 0.70 (g/ml),
   wherein the heavy liquefied gas is hydrofluoroolefin, and
   wherein the aerosol composition is obtained by emulsifying the aqueous concentrate, the hydrofluoroolefin and the light liquefied gas.

2. The aerosol composition of claim 1, wherein a content of the heavy liquefied gas (a) in the liquefied gas is 5 wt% or more.

3. The aerosol composition of any of claim 1 or 2, wherein the liquid density at 20°C of the aqueous concentrate is from 0.90 to 1.10 (g/ml).

4. The aerosol composition of claim 1, wherein when the aerosol composition is sprayed, sherbet frozen at least partly is formed, and when spraying the composition against a 5 cm high flame located apart by 15 cm, elongation of the flame is 50 cm or less.

5. The aerosol composition of claim 1, wherein foams emitting a foam-cracking sound are formed when the aerosol composition is sprayed, and a height of a flame when the foams are ignited is 35 cm or less.

6. The aerosol composition of claim 1, wherein many independent foams in the form of soap bubble are formed when the aerosol composition is sprayed, and a falling speed of the foams is from 0.015 to 0.2 m/sec.

7. The aerosol composition of claim 6, wherein the aqueous concentrate comprises an ionic surfactant and/or an ionic resin.

**Patentansprüche**

1. Eine Aerosolzusammensetzung, die umfasst:

   10 bis 60 Gewichts-% eines wässrigen Konzentrats, das ein Tensid und Wasser und
   40 bis 90 Gewichts-% eines verflüssigten Gases umfasst, und

   durch das Emulgieren des wässrigen Konzentrats und des verflüssigten Gases erhalten wird,
   wobei der Gehalt von Wasser in dem wässrigen Konzentrat 40 bis 99 Gewichts-% beträgt,
   wobei das verflüssigte Gas ein schweres verflüssigtes Gas (a), das eine Flüssigkeitsdichte bei 20 °C von 1,15 bis 1,30 (g/ml) aufweist, und ein leichtes verflüssigtes Gas (b), das eine Flüssigkeitsdichte bei 20 °C von 0,50 bis 0,70 (g/ml) aufweist, umfasst,
   wobei das schwere verflüssigte Gas ein Hydrofluoroolefin ist, und
   wobei die Aerosolzusammensetzung durch das Emulgieren des wässrigen Konzentrats, des Hydrofluoroolefins und des leichten verflüssigten Gases erhalten wird.

2. Die Aerosolzusammensetzung des Anspruch 1, wobei der Gehalt des schweren verflüssigten Gases (a) in dem verflüssigten Gas 5 Gewichts-% oder mehr beträgt.

3. Die Aerosolzusammensetzung gemäß einem der Ansprüche 1 oder 2, wobei die Flüssigkeitsdichte bei 20 °C des wässrigen Konzentrats von 0,90 bis 1,10 (g/ml) beträgt.

4. Die Aerosolzusammensetzung gemäß Anspruch 1, wobei, wenn die Aerosolzusammensetzung versprüht wird, zumindest zum Teil gefrorenes Sorbet gebildet wird, und die Verlängerung der Flamme 50 cm oder weniger beträgt wenn die Zusammensetzung gegen eine 5 cm hohe Flamme, die sich 15 cm entfernt befindet, gesprüht wird.

5. Die Aerosolzusammensetzung gemäß Anspruch 1, wobei beim Sprühen der AerosolZusammensetzung Schäume gebildet werden, die ein Schaum-knackendes Geräusch emittieren, und die Höhe der Flamme 35 cm oder weniger beträgt, wenn die Schäume entzündet werden.

6. Die Aerosolzusammensetzung gemäß Anspruch 1, wobei beim Sprühen der AerosolZusammensetzung viele unabhängige Schäume in der Form von Seifenblasen gebildet werden, und die Fallgeschwindigkeit der Schäume von 0,015 bis 0,2 m/sec beträgt.

7. Die Aerosolzusammensetzung gemäß Anspruch 6, wobei das wässrige Konzentrat ein ionisches Tensid und/oder ein ionisches Harz umfasst.

**Revendications**

1. Composition d'aérosol comprenant :

   10 à 60 % en poids d'un concentré aqueux comprenant un tensioactif et de l'eau, et
   40 à 90 % en poids d'un gaz liquéfié, et

   obtenue par émulsification du concentré aqueux et du gaz liquéfié, dans laquelle une teneur en eau dans le concentré aqueux est de 40 à 99 % en poids, dans laquelle le gaz liquéfié comprend un gaz liquéfié lourd (a) ayant une densité de liquide à 20°C de 1,15 à 1,30 (g/ml) et un gaz liquéfié léger (b) ayant une densité de liquide à 20°C de 0,50 à 0,70 (g/ml), dans laquelle le gaz liquéfié lourd est de l'hydrofluoro-oléfine, et
   dans lequel la composition d'aérosol est obtenue par émulsification du concentré aqueux, de l'hydrofluoro-oléfine et du gaz liquéfié léger.

2. Composition d'aérosol selon la revendication 1, dans laquelle une teneur en gaz liquéfié lourd (a) dans le gaz liquéfié est de 5 % en poids ou plus.

3. Composition d'aérosol selon l'une quelconque des revendications 1 ou 2, dans laquelle la densité de liquide à 20°C du concentré aqueux est comprise entre 0,90 et 1,10 (g/ml).

4. Composition d'aérosol selon la revendication 1, dans laquelle lorsque la composition aérosol est pulvérisée, une glace au moins partiellement congelée est formée, et lors de la pulvérisation de la composition contre une flamme de 5 cm de haut espacée de 15 cm, l'allongement de la flamme est de 50 cm ou moins.

5. Composition d'aérosol selon la revendication 1, dans laquelle des mousses émettant un son de fissuration de mousse sont formées lorsque la composition aérosol est pulvérisée, et une hauteur de flamme lorsque les mousses sont enflammées est de 35 cm ou moins.

6. Composition d'aérosol selon la revendication 1, dans laquelle de nombreuses mousses indépendantes sous la forme de bulles de savon sont formées lorsque la composition d'aérosol est pulvérisée, et une vitesse de chute des mousses est comprise entre 0,015 et 0,2 m/s.

7. Composition d'aérosol selon la revendication 6, dans laquelle le concentré aqueux comprend un tensioactif ionique et/ou une résine ionique.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3439672 B
- JP 4173034 B
- JP 4098093 B
- US 2006269484 A1
- JP 2255890 A
- JP 2009227662 A
- EP 2245927 A1